Europäisches Patentamt

European Patent Office

Office européen des brevets

(11) Publication number: **0 256 708**
**A2**

(12) **EUROPEAN PATENT APPLICATION**

(21) Application number: **87306692.2**

(22) Date of filing: **29.07.87**

(51) Int. Cl.4: **A61C 8/00**

(30) Priority: **13.08.86 US 896524**
**13.08.86 US 896101**
**20.10.86 US 920796**
**20.10.86 US 920781**
**25.11.86 US 934651**
**25.11.86 US 934638**
**29.12.86 US 947176**

(43) Date of publication of application:
**24.02.88 Bulletin 88/08**

(84) Designated Contracting States:
**AT BE CH DE ES FR GB GR IT LI LU NL SE**

(71) Applicant: **ROSS SYSTEMS CORPORATION**
**324 Datura Street Suite 201**
**West Palm Beach Florida 33401(US)**

(72) Inventor: **Ross, Stanley E.**
**7170 Melbourne Lane**
**Boca Raton Florida 33434(US)**

(74) Representative: **Davies, Christopher Robert et al**
**Frank B. Dehn & Co. Imperial House 15-19 Kingsway**
**London WC2B 6UZ(GB)**

(54) **Process and apparatus for forming a dental prosthesis and installing same in a patient's mouth.**

(57) A dental anchor (10 ) is adapted to be installed in a predrilled bore formed in jaw bone tissue for retaining a dental prosthesis, the anchor comprising a generally cylindrical body (12) defining a longitudinal axis, the body being open at a front end thereof and including a cylindrical front cavity (14) communicating with the open front end for receiving a cylindrical core. The front cavity is surrounded by a front portion of said body, the front portion being apertured (24) to accommodate the growth of bone tissue therethrough, and the body being open at a non-apertured rear portion thereof and including a rear cavity (16). The rear cavity is internally threaded to receive externally threaded prosthesis-attachment means (80) and the front and rear portions of said body include front and rear non-threaded outer peripheries (52 and 38), respectively, which are of the same diameter and define a maximum outer diameter of said anchor. Other aspects of the invention relate to a method of implanting the anchor, methods of forming an associated dental prosthesis, methods of drilling the bore in the jaw, and a method of selecting an appropriate dental anchor.

FIG. 2

## PROCESS AND APPARATUS FOR FORMING A DENTAL PROSTHESIS AND INSTALLING SAME IN A PATIENT'S MOUTH

### BACKGROUND OF THE INVENTION

The present invention relates to the securement of dental anchors in dental bone tissue to secure dental prostheses within the mouth.

### Anchor and Securement Thereof

It has been proposed to mount dental prostheses within a patient's mouth by affixing the prostheses to dental anchors which are embedded directly within the jaw bone. The anchors and embedding techniques heretofore proposed and utilized in practice have not met with universal acceptance among dental practitioners due to considerable limitations regarding the placement of the anchors within the mouth and/or uncertainties as to the useful lifespan of the anchors.

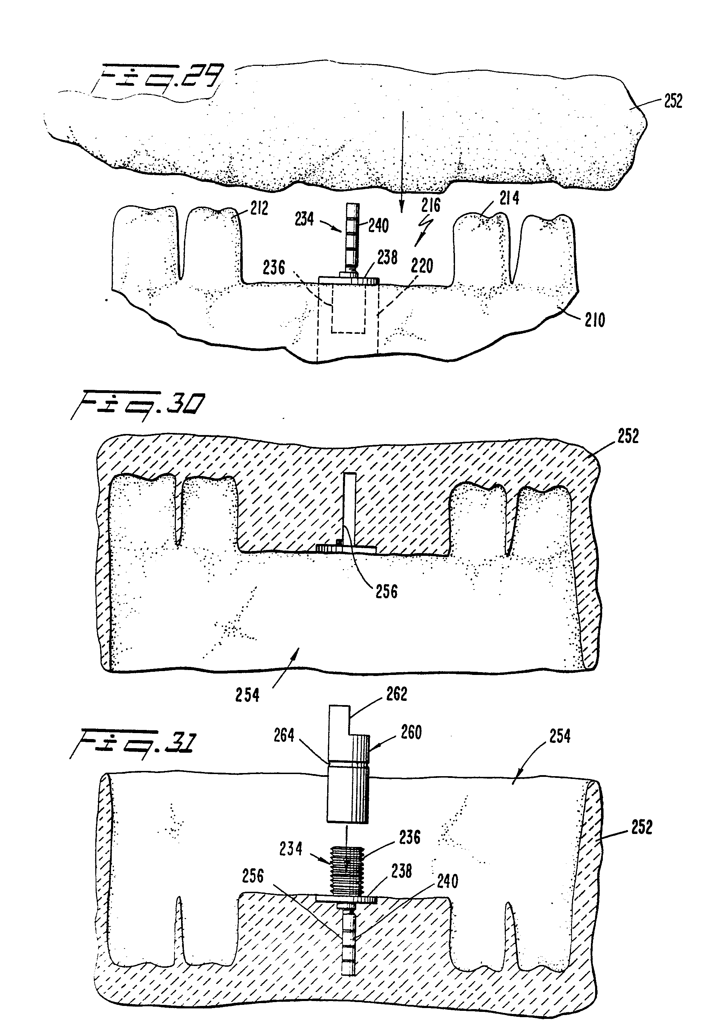

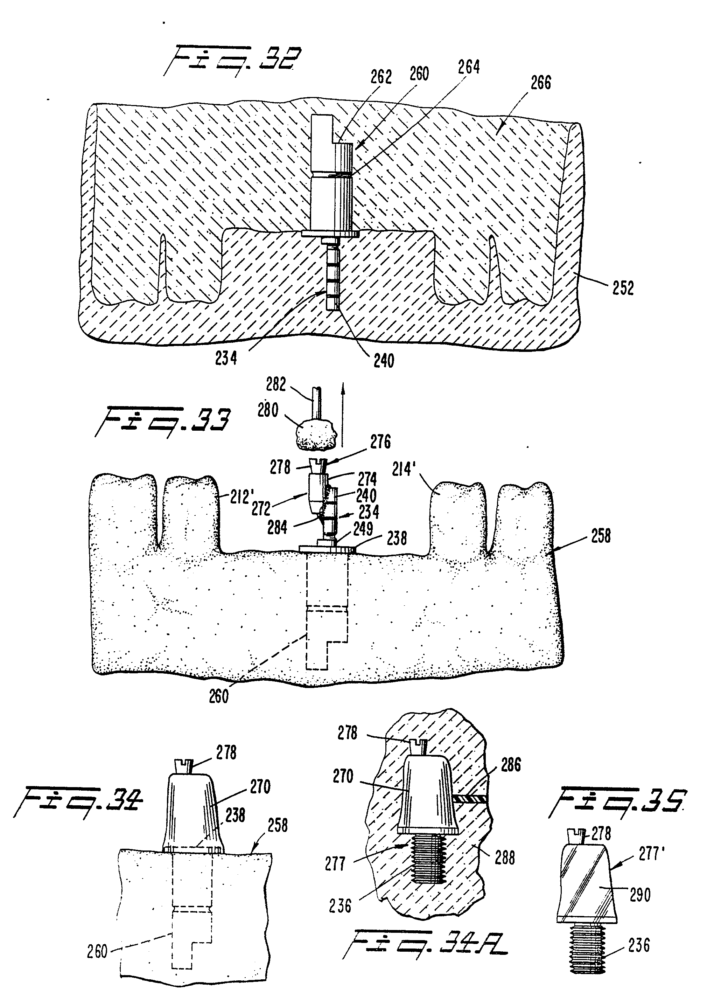

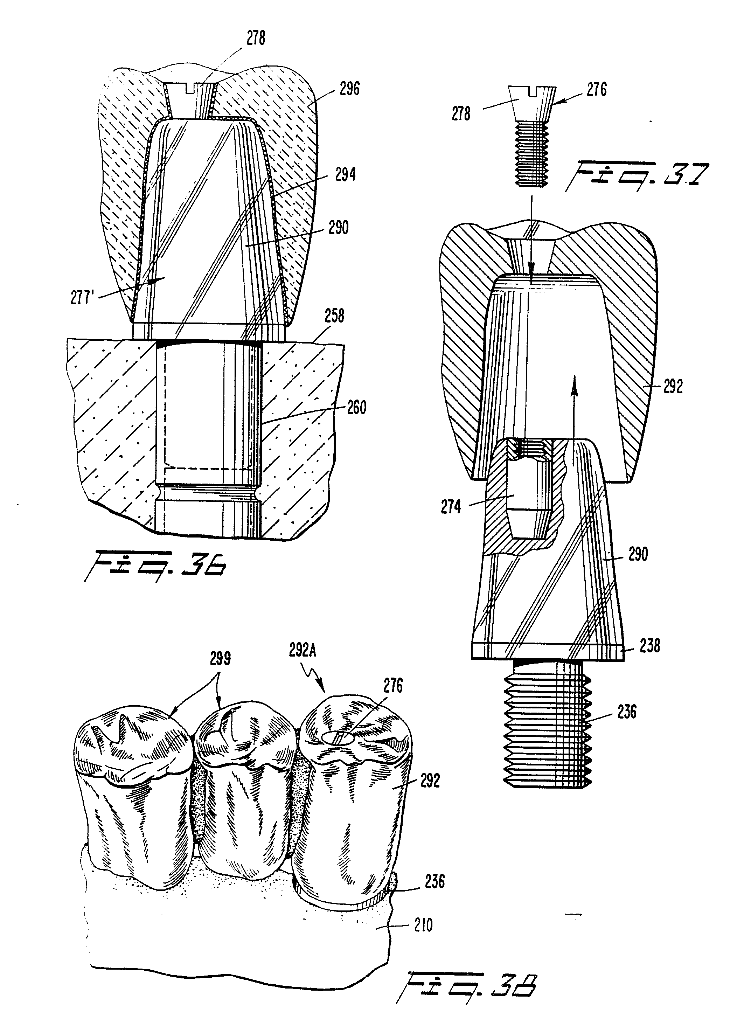

In that regard, one currently employed technique involves the use of an anchor 1 (depicted in Figs. 11-13 of the accompanying drawings) having external threads 2 by means of which the anchor is to be screwed into the bone tissue 3. A bore is first drilled into the bone tissue, the bore having a diameter less than the maximum diameter of the threads of the anchor. By screwing the insert 1 into the bore, the threads 2 cut into the bone tissue 3. Such a technique is disclosed in U.S. Patent No. 4,431,416.

However, such a technique can result in so-called "saucerization" wherein some of the bone tissue does not grow back against the anchor, i.e., wherein areas between the bone and implant are occupied by soft tissue 4 which does not contribute appreciably to the securement of the anchor. In cases where anchors have been installed in the lower jaw bone, saucerization has been observed to occur at the upper end of the bore. Such saucerized areas can become infected, leading to a spreading of the saucerization along the anchor whereby the securement of the anchor is significantly undermined.

Although the exact causes of saucerization are not yet known for certain, it is the belief of the present inventor that because of the harsh treatment to which the bone is subjected during anchor installation, the rate at which the bone is able to regenerate and grow against the anchor is deterred to such an extent that soft gum tissue 4 is able to grow into and occupy the space between the anchor 1 and the bone 3 at the open end of the bore. That deterred growth rate may result from a number of factors, including the severe traumatizing of the bone as it is cut and fractured along the region 3A by the self-tapping threads of the anchor, and/or the likelihood that the bone is deprived of blood at least at that portion of the bone located at the top of a bore in the lower jaw since blood tends to gravitate away from that bone portion. In that regard, it is well known that blood, once it clots, will develop fibroblasts and osteoblasts which promote the development of bone tissue. By depriving the bone tissue of an ample blood supply, the regeneration and regrowth of bone tissue will be retarded, thereby permitting soft gum tissue to enter the area between the anchor and bone. If such saucerization occurs and spreads along the length of the anchor, the lifespan of the anchor can be significantly shortened.

Some dental practitioners install more than the required number of anchors into the bone, whereby the extra anchors are kept available as "spares" in the event that the active anchor(s) becomes dislodged from the bone. Besides being more expensive and inconvenient to the patient, that practice is only feasible in cases involving the installation of a relatively long prosthesis wherein the space between the active anchor(s) is large enough to accommodate the "spare" anchors.

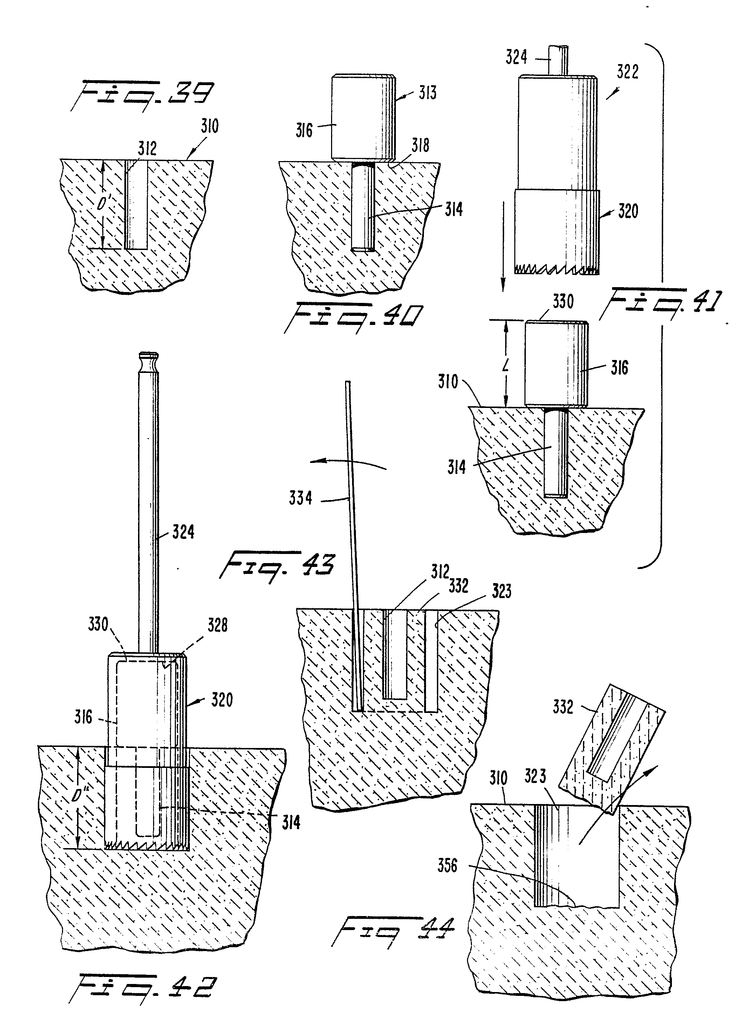

In order to augment the securement of the anchor, the anchor can be provided with a hollow front end which receives a core 5 of the bone (FIG. 12). Such an expedient involves the cutting of an annular kerf in the bone tissue to leave a cylindrical core of bone surrounded by the kerf. The open front end of the anchor receives the core as the anchor is inserted into the kerf. The wall of the anchor surrounding the core is provided with holes 6 to enable bone tissue to grow through the holes. In the case of anchors which include self-tapping screw threads 2, however, the screwing-in of the anchor may impose sufficient lateral stresses on the core to cause the core to be broken-off at its base. Even if the core is eventually able to regenerate and regrow, the overall anchoring process could be delayed.

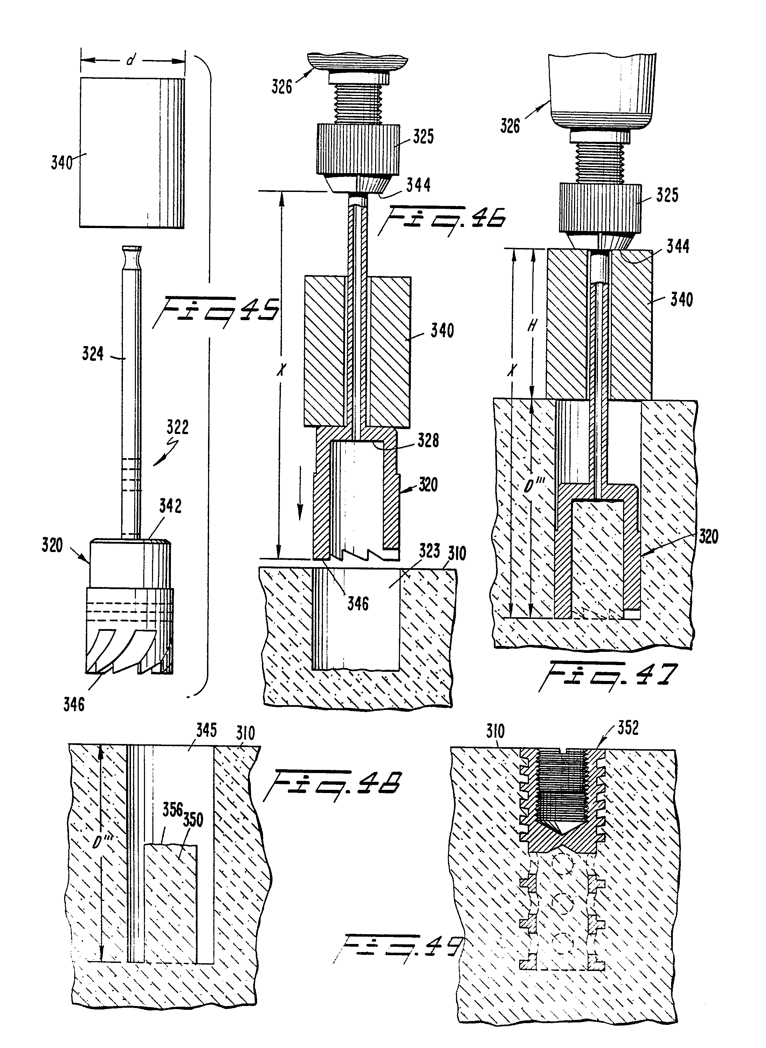

Furthermore, the act of screwing-in a self-tapping anchor requires substantial torque imposed by means of a ratchet wrench which mates with a wrench-receiving socket 7 of the anchor (FIG. 13). However, such wrenches are relatively large and cumbersome and require a relatively large amount of space to accommodate tool manipulation. Consequently, such screw-in anchors are not suitable for use in the back portions of a patient's mouth where there exists little room to turn the handle of the wrench, or in narrow areas between existing teeth which are not large enough to accommodate the head of the wrench.

In addition, the fracturing of bone tissue caused by the screw threads renders the anchors unsuitable for use in areas of exceptionally thin bone. Therefore, in cases where a patient's bone has been diminished in thickness due to surgical operation, injury, etc., the use of a screw-in type anchor may not be feasible.

Therefore, it will be appreciated that for at least the above-discussed reasons, the overall utility and versatility of screw-in type anchors has been significantly limited.

It has also been proposed to pre-form female screw threads in the bone by means of a thread-forming drill, rather than by the use of self-tapping threads on the anchor. Such a technique is disclosed in an article entitled "A 15-Year Study of Osseointegrated Implants in the Treatment of the Edentulous Jaw" by Adell, Lekholm, Rockler, and Branemark, published in the International Journal of Oral Surgery, Munksgaard, Copenhagen, 1981, Vol. 10, pp. 387-416. In that technique, the thread-forming drill does not leave a core in the bore to augment the anchoring action. Also, the drill is designed to form a widening at the mouth of the bore to accommodate the insertion of an anchor having an enlarged collar at is outer end. In practice, saucerization results from the formation of such a widening, and the saucerization gradually spreads, e.g., by about 0.10 mm per year along the length of the anchor according to the above-referenced article. Once a sufficient length of saucerization has occurred, the anchor will be inadequately secured. It should further be noted that even though the female threads are predrilled in the bore, the threads on the anchor are intended to be partly self-tapping. Thus, a wrench is needed, whereby the areas of the mouth in which that anchor can be employed are limited, and a certain amount of bone fracture and bone trauma will likely occur during installation of the anchor.

Attachment of Prosthesis to Anchor

One method of attaching a prosthesis to a secured anchor involves cementing a pillar within an open recess of the anchor such that a snap socket on the end of the pillar is adapted to receive a mating part of the prosthesis. The pillar can be bent in order to achieve a certain degree of parallelism relative to the adjacent teeth. However, the bending of the pillar may result in a cracking of the pillar, and, thus, the extent to which the pillar can be bent is limited. If proper parallelism and jaw-to-jaw height are not achieved, the teeth will not correctly occlude. Furthermore, unless the prosthesis has an easily replaceable crown, the prosthesis cannot be easily repaired in the event of damage or wear thereof.

Instead of inserting a bendable pillar into the anchor, a plastic pillar could be inserted into the anchor which is then cut and shaped while in the patient's mouth in an attempt to achieve parallelism with adjacent teeth. However, the equipment necessary to achieve a precise degree of parallelism will not fit into a patient's mouth and thus the chances of achieving exact parallelism are reduced. After being shaped, the pillar could be inserted into a conventional investment-type casting apparatus which melts the plastic and replaces it with a permanent material such as gold.

A brochure presented by Metaux Precieux SA Metalor, describes steps for fabricating a prosthesis which is to be inserted into a tooth cavity. Wax is inserted into the tooth cavity to form a wax model. The wax model is removed from the tooth cavity, and an internally threaded sleeve is pushed directly into the wax body. Then, the sleeve is removed from the wax body and is replaced by a positioner which occupies the hole made by the sleeve. The wax body is then subjected to a conventional investment-type casting procedure wherein the wax is melted-out and replaced by a permanent material such as gold. Then, the positioner is removed and replaced by the sleeve which is soldered in place within the gold body. A screw is threaded into the sleeve and a release material is applied to the gold body followed by the application of a wax layer which conforms to the desired tooth shape. Following an investment-type casting operation, the wax is replaced by a material such as gold to form a crown or overlay which is removable from the original gold body, due to the release material. The original gold body is cemented into the tooth cavity, and the overlay is able to be replaced by removal of the screw. This procedure is very time-consuming due especially to the need for soldering the sleeve into the gold body. Also, the pushing of the sleeve into the wax body can deform the wax body from its intended shape. The overall procedure is not well suited to the creation of a prosthetic tooth to be installed in an anchor attached within a patient's jaw.

A procedure for forming a dental prosthesis to be mounted in an anchor has been proposed by Interpore International of Irvine, California. That procedure involves the implanting of an anchor in a patient's jaw, the anchor having a threaded hole which opens into the interior of the patient's mouth. Into that hole is inserted a threaded stem of a plastic impression pin such that a post of the pin projects beyond the jaw and into the mouth. A clay impression of the dental arch is then made. The impression pin is then unscrewed from the anchor and the post is inserted into the respective cavity of the impression, leaving the threaded stem exposed. A dowel is threaded onto the stem, and a dental model is formed by pouring dental stone into the impression. The dowel becomes permanently embedded within the model. The impression pin is then unscrewed from the dowel and replaced by a titanium element (i.e., a so-called IME). The IME includes an internally threaded hole and an externally threaded stem which is to be threaded into the dowel (and eventually into the anchor once a prosthesis has been formed on the IME). A titanium coronal screw is threaded into the internally threaded hole so as to project a predetermined distance out of the hole. A sleeve is applied around the screw and is waxed-up so as to form a shaped wax body. The unit is then subjected to a conventional investment-type casting step wherein the wax is replaced by a suitable permanent material such as gold. That gold body can be removed from the IME by removing the screw. A suitable porcelain layer can be baked onto the gold body. With such a procedure, it is very difficult to achieve proper parallelism between the prosthesis and the adjacent teeth, as well as a precise jaw-to-jaw height, because of the inability to bend or cut the screw. As a result, the permanent material is mounted so as to be movable relative to the screw in an attempt to enable the permanent material to "float" and become self-aligned with the opposing teeth of the other jaw. Another method for forming and installing a dental prosthesis is described in an article by R. Adell et al entitled "A 15-year Study of Osseointegrated Implants in the Treatment of the Edentulous Jaw", printed in the International Journal of Oral Surgery, 1981, Vol. 10, pp. 387-416. As described in that article, a partially self-tapping anchor is threaded into a predrilled, partially pretapped hole in patient's jaw. The anchor includes a threaded hole opening into the patient's mouth. After the healing process, an enlarged abutment is attached to the anchor by means of a first screw which passes through the abutment and enters the threaded hole of the anchor. A bridge is prepared in conventional fashion and is attached to the abutment by means of a second screw which passes through the bridge and enters a threaded hole in the head of the first screw. With such a procedure it is difficult to achieve a proper parallelism between the teeth of the bridge and adjacent teeth, as well as proper jaw-to-jaw height. Moreover, since the bridge rests upon the abutment, gaps are created between the bridge and the gum which are undesirable from an esthetics and phonetics standpoint.

## Drilling of Bore in Bone Tissue

The bore in which the anchor is secured is conventionally drilled in three stages. In the first stage, a pilot hole is made by a drill bit. Then, within the pilot hole is installed a guide bushing which comprises two cylinders attached end-to-end. One of the cylinders is of small diameter and is inserted into the pilot hole. The drill head of a trephine drill is inserted over the large diameter cylinder in telescoping fashion and is advanced into the bone tissue during a second stage of the operation. The large diameter cylinder acts as a guide for the trephine drill to prevent the latter from moving sideways. The drill is advanced to a depth short of the final depth and, when removed, leaves a center core of bone. A hand tool is then inserted into the bore in order to break-off the core. Then, during the final step, the drill is reinserted into the bore and advanced to a final depth.

It is necessary that the bore be made sufficiently deep to receive the anchor, but not so deep that a nerve may be cut and damaged. This requires a certain drilling precision which, to date, has been difficult to achieve.

At present, it is conventional to employ a trephine drill which has markings on its side to indicate the depth attained by the drill. However, it is difficult for an operator to accurately see the markings during a drilling procedure, requiring the operator to repeatedly interrupt the drilling procedure to check the marks. Such a practice is time-consuming and does not guarantee that the required depth will not be exceeded.

It has been proposed in Swiss Patent No. 302,233 to terminate the advancement of a drill by means of a stop adjustably fixed on the drill. However, it is difficult and time-consuming to adjust the stop to a precise position on the drill. Also, since the stop is fixed by a set screw, it is possible that if the set screw becomes inadvertently loosened, allowing the stop to move, severe damage can result from excessive advancement into the jaw bone.

It would be desirable to provide drilling apparatus and methods which enable a bore to be drilled quickly to the desired depth, with absolutely no chance whatsoever that the desired depth could be exceeded to any appreciable extent.

## Drill Structure

Conventional dental trephine drills do not easily cut through jaw bone tissue; drilling operations conducted with such drills are tedious and time-consuming, adding to the discomfort of the patient. Also, the drills might leave burrs on the wall of the bore which resist the entry of the anchor. To remove burrs, it has been necessary to repeatedly reciprocate the drill within the bore. Such a practice is time-consuming, increases the discomfort of the patient and may result in the bore being made of larger diameter than is desired for optimal results. That is, it is desirable to enable the outer surface of the anchor to contact the wall of the bore in order to promote the occurrence of blood clotting and the retention of blood clots in the vicinity of the entire wall of the bore so that the regeneration of the bone tissue is accelerated. The attainment of this goal can be impaired if the bore diameter is excessively enlarged as the result of repeated reciprocation of the drill when the bore is being drilled.

Another concern during the drilling of bores in bone tissue relates to the fact that the bone tissue can be damaged by high temperature build-ups occurring as the result of frictional contact with the drill. Such temperature-induced damage can adversely affect the ability of the bone tissue to regenerate itself and grow against the anchor. Conventional trephine drills include a central passage for conducting fluid such as a saline solution or sterile water to the drill head intended for cooling the bone tissue and the drill head as well as flushing cuttings from the bore. However, as the bone core enters the front opening of the drill head, the flow of fluid is obstructed, whereby the benefits which would be otherwise attributable to the fluid flow are minimized. Also, such blockage of fluid flow creates a fluid pressure build-up which resists the advancement of the drill head.

Selection of Proper Anchor

It is important that an anchor of proper size (i.e., proper diameter and length) be selected. Various factors influence the size selection of the anchor, such as the amount of space at the anchor site between existing teeth, the width (thickness) of the patient's jaw bone at the anchor site, the depth of the jaw bone, and the proximity of nerves which must be avoided during the drilling procedure.

One present practice for the selection of anchors involves the use of a transparent sheet which contains pictorial reproductions of anchors of various size. That sheet is placed in an X-ray view box along with a patient's X-ray, and the various pictorial reproductions of anchors on the sheet are sequentially placed in overlying relationship to the anchor site on the X-ray, until a proper anchor size is determined. The pictorial reproductions of the anchors and the X-ray are of the same magnification. Presently available sheets of this type carry pictorial representations of anchors which are at 25 percent magnification and are instructed to be used in conjunction with X-rays which are at 25 percent magnification.

Such a practice requires a considerable amount of skill and care since the transparent sheet includes indicia in addition to the reproductions of the anchors which can interfere with a full clear viewing of the X-ray. Also, after the procedure is performed, there remains no permanent pictorial record which can be re-checked and verified at a later period prior to the installation of the anchor into the patient's jaw bone or which can be compared to a later X-ray after the anchor installation procedure has been completed.

It is, therefore, an object of respective aspects of the present invention to minimize or obviate problems of the types discussed above.

This object is achieved by various different aspects of the invention as defined by the appended claims.

Certain embodiments of the invention will now be described, by way of example only, with reference to the accompanying drawings, wherein:

FIGURE 1 is a side elevational view of a first embodiment of a dental anchor according to the present invention;

FIGURE 2 is a longitudinal sectional view taken through the anchor of FIG. 1 as the anchor is being advanced toward a predrilled bore in bone tissue;

FIGURE 3 is a view similar to FIG. 2 after the anchor has been pushed into the predrilled bore;

FIGURE 4 is a view similar to FIG. 3 with a prosthesis attachment threadedly mounted in the anchor;

FIGURE 5 is a view similar to FIG. 3 with a healing screw threadedly mounted in the anchor;

FIGURES 6 and 7 are schematic views representing steps involved in installing the anchor in bone tissue;

FIGURE 8 is a side elevational view of a second embodiment of a dental anchor according to the present invention;

FIGURE 9 is a longitudinal sectional view taken through the anchor of FIG. 8;

FIGURE 10 is a view similar to FIG. 9 after the anchor has been pushed into a pre-drilled bore, and a prosthesis attachment is depicted in broken lines;

FIGURE 11 is a side elevational view of a prior art anchor;

FIGURE 12 is a cross-sectional view of the FIG. 11 anchor after it has been screwed into a jaw bone;

FIGURE 13 is a top view of the FIG. 12 anchor;

FIGURE 14 is a side elevational view of a reservoir-containing anchor according to the present invention;

FIGURE 15 is a longitudinal sectional view taken through the anchor of FIG. 14 depicting the anchor as it is being inserted into a bore formed in jaw bone tissue;

FIGURE 16 is a view similar to FIG. 15 after the anchor has been pushed into a predrilled bore;

FIGURE 17 is an enlarged fragmentary view of the anchor of FIG. 14 depicting the recessed nature of the rear surfaces of the ribs;

FIGURES 18 and 19 are schematic views representing steps involved in installing the anchor of FIG. 14;

FIGURE 20 is a view similar to FIG. 16 with the anchor containing a prosthesis-retaining member;

FIGURE 21 is a view similar to FIG. 20 with the anchor containing a healing screw;

FIGURE 22 is a view of the anchor in place between a pair of teeth in the jaw;

FIGURE 23 is a view similar to FIG. 16 of a modified form of reservoir-containing anchor suitable for use in upper jaw bones;

FIGURE 24 is a view similar to FIG. 17 of yet another rib configuration;

FIGURE 25 is a vertical sectional view taken through a bore formed in a jaw bone of a patient;

FIGURE 26 is a view similar to FIG. 25 after an anchor has been pushed into the bore;

FIGURE 27 is a view similar to FIG. 26 after a healing screw has been inserted into the anchor, and the bone tissue has grown through openings in the anchor;

FIGURE 28 is a view similar to FIG. 26 after the healing screw has been removed and a pin according to the present invention has been threaded into the anchor;

FIGURE 28A is a longitudinal end view of the pin depicted in FIG. 28;

FIGURE 29 is an elevational view of a portion of a dental arch with the pin installed therein, as a piece of clay or dental impression material is being pressed onto the arch;

FIGURE 30 is a view of the clay after an impression has been made and the clay has been removed from the dental arch;

FIGURE 31 is a view of the clay impression after it has been inverted and the pin has been reinserted therein, and a protective sheath is in the process of being installed onto a threaded stem portion of the pin;

FIGURE 32 is a view similar to FIG. 31 after the sheath has been installed and plaster has been poured into the clay impression;

FIGURE 33 is an elevational view of the plaster model just after a fastener assembly has been attached to a post portion of the pin;

FIGURE 34 is an elevational view of the pin, still mounted in the plaster model, after the pin has been waxed-up;

FIGURE 34A is a view of a preliminary unit comprising the pin, the fastener assembly, and the wax, disposed in an investment material prior to carrying out an investment-type casting operation;

FIGURE 35 is an elevational view of the unit after the investment-type casting operation wherein the wax has been replaced by a permanent material to form a secondary unit;

FIGURE 36 is an elevational view of the secondary unit mounted in the plaster model, with layers of release material and wax disposed on the body of permanent material;

FIGURE 37 is an exploded view of the secondary unit and an overlay, with a screw of the fastener assembly in a removed condition;

FIGURE 38 is a perspective view of a bridge attached to a jaw bone in accordance with the present invention;

FIGURE 39 is a cross-sectional view taken through the jaw bone of a patient after a pilot hole has been drilled therein;

FIGURE 40 is a view similar to FIG. 39 after a guide bushing has been inserted into the pilot hole;

FIGURE 41 is a view similar to FIG. 40 as the head of a trephine drill is being installed onto a large cylindrical portion of the bushing;

FIGURE 42 is a view similar to FIG. 41 after the trephine drill has advanced into the bone tissue to form an initial bore therein and has contacted an end of the large diameter portion of the bushing;

FIGURE 43 is a sectional view taken through the jaw bone depicting the initial bore which has been formed, as a tool is in the process of breaking off a core portion of the initial portion;

FIGURE 44 is a view similar to FIG. 43 as the broken core portion is being removed from the initial bore;

FIGURE 45 is an exploded side elevational view of a trephine drill and a stop which is to be installed thereon;

FIGURE 46 is a view of the trephine drill, with the stop mounted thereon, the drill being mounted in the chuck of a drill motor and is being advanced into the initial bore;

FIGURE 47 is a view similar to FIG. 46 after the drill head has been advanced sufficiently far to form a final bore;

FIGURE 48 is a cross-sectional view through the jaw bone depicting the final bore;

FIGURE 49 is a sectional view through the jaw bone after a dental anchor has been installed in the final bore, and the bone tissue has grown against the dental anchor;

FIGURE 50 is a side elevational view of a dental trephine drill according to the present invention;

FIGURE 51 is a front end view of the trephine drill;

FIGURE 52 is a cross-sectional view taken along the line 52-52 in FIG. 50;

FIGURE 53 is a cross-sectional view taken along the line 53-53 in FIG. 50;

FIGURE 54 is a longitudinal sectional view taken through the drill after the drill has cut an initial bore in bone tissue;

FIGURE 55 is similar to FIG. 54 after the initially formed core has been removed and the drill has been further advanced to extend the bore;

FIGURE 56 is a longitudinal sectional view through the bore after the drill has been removed;

FIGURE 57 is a longitudinal sectional view taken through the bore and through an anchor which has been inserted into the bore, after the bone tissue has grown against the anchor;

FIGURE 58 is a fragmentary plan view of a transparent transfer sheet according to the present invention;

FIGURE 59 is a plan view of a portion of the transfer sheet positioned in superimposed relationship to an X-ray as a pictorial representation is being rubbed off the transfer sheet and onto the X-ray;

FIGURE 60 is a plan view of the X-ray after the pictorial representation has been attached thereto;

FIGURE 61 is a view similar to FIG. 50 as the pictorial representation is being removed from the X-ray;

FIGURE 62 is a view similar to FIG. 59 as a different pictorial representation is being transferred to the X-ray; and

FIGURE 63 is a view similar to FIG. 61 after the different pictorial representation has been transferred to the X-ray.

## DETAILED DESCRIPTION OF PREFERRED EMBODIMENTS OF THE INVENTION

### Anchor and Securement Thereof

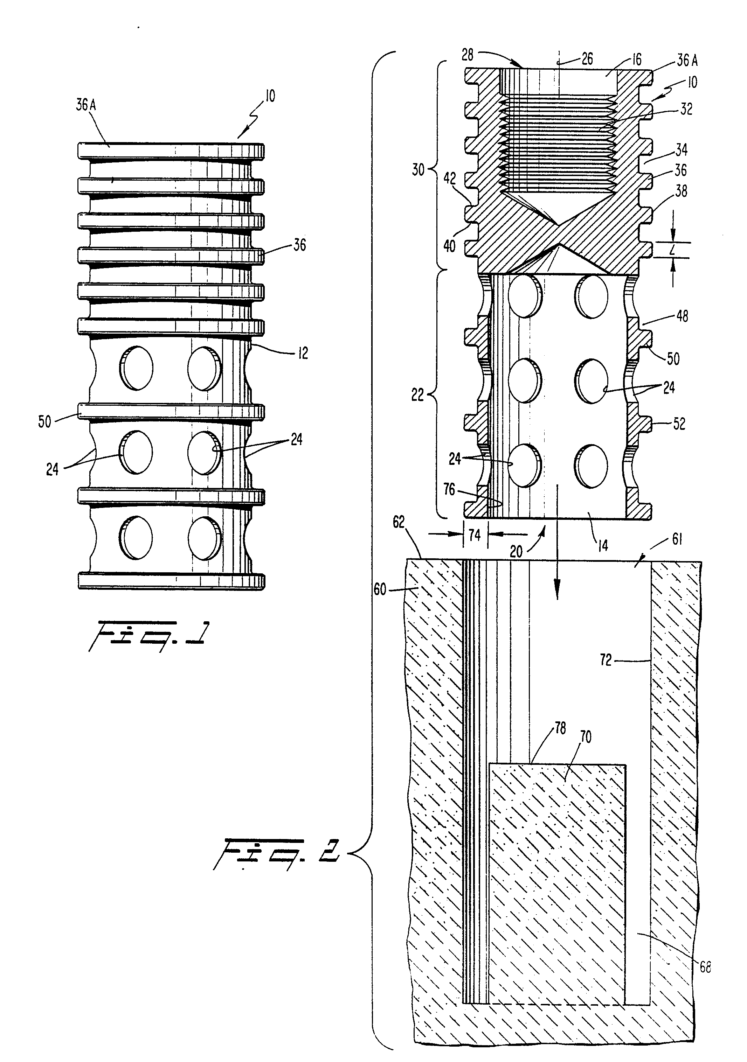

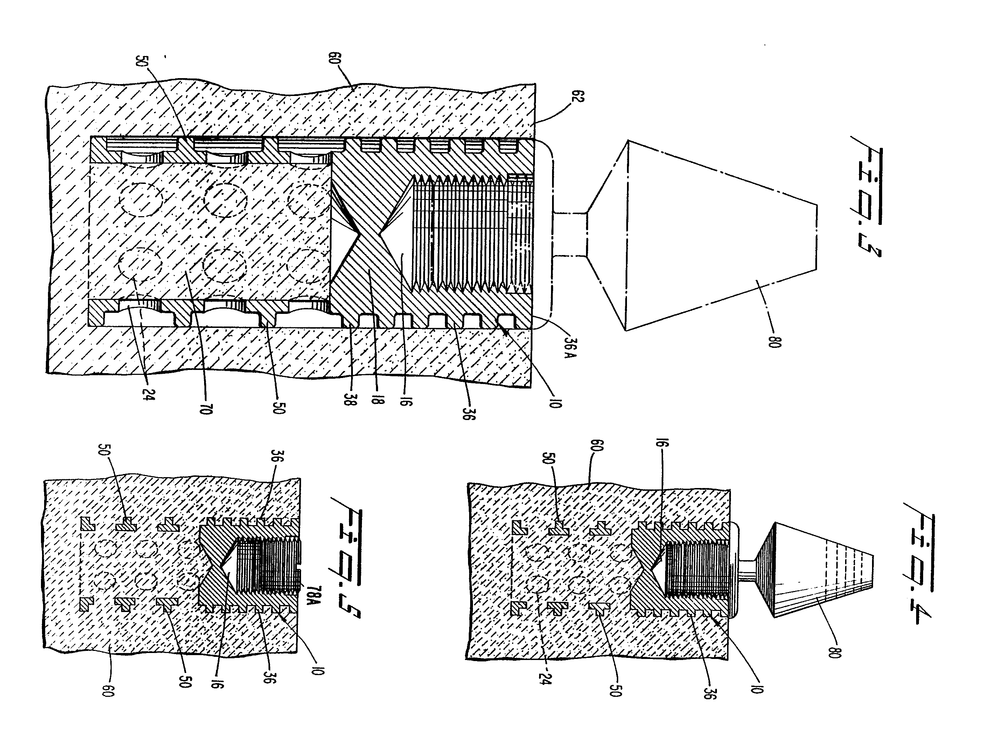

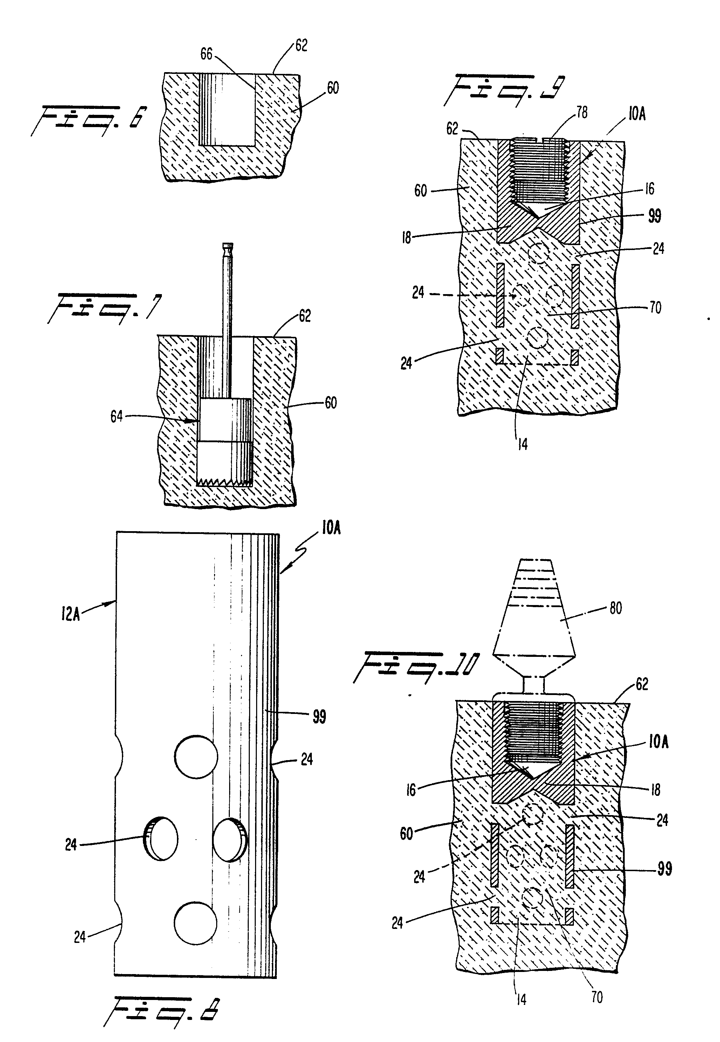

A dental anchor or implant 10 according to the present invention, depicted in FIGS. 1-8, is suitable for use in lower jaw bones. The anchor comprises a generally cylindrical body 12 formed of a biocompatible material such as, for example, titanium such as commercially pure titanium or a titanium alloy such as Ti 6AL 4V, or other equivalent materials as will be apparent to those skilled in the art. The body defines front and rear circular cylindrical cavities 14, 16 which are separated by a divider wall 18 (FIG. 3). The front cavity 14 is open toward a front end 20 of the body 12 and is surrounded by a front portion 22 of the body. That front portion 22 is apertured by means of a plurality of through-holes 24. The through-holes are arranged in circumferential rows, each row being spaced apart in a direction parallel to the longitudinal axis 26 of the body.

The rear cavity 16 opens toward a rear end 28 of the body 12 and is surrounded by a rear portion 30 of the body. That rear portion 30 is non-apertured, i.e., it does not afford any appreciable external communication for the rear cavity except via the rear opening 28. The rear cavity includes an internal wall which contains female screw threads 32.

The exterior of the rear portion 30 comprises a plurality of longitudinally alternating grooves 34 and ribs 36. The ribs 36 are circular and extend circumferentially continuously around the body 12 in a plane oriented perpendicularly relative to the axis 26. Each rib includes an outer surface 38 which is smooth and extends circumferentially continuously. That is, the surface 38 is circular as opposed to being helical as in the case of screw threads. The surface 38 also has a dimension L in the longitudinal direction. The surface 38 is flat in the longitudinal direction although the front and rear edges of the surface are slightly radiused to eliminate sharp corners. The outer surfaces 38 are of equal outer diameter and define the outer periphery of the rear portion 30.

Each rib includes front-facing and rear-facing surfaces 40, 42 which are circumferentially continuous and oriented perpendicularly relative to the axis 26.

The exterior of the front portion 22 of the. body 12 includes longitudinally alternating grooves 48 and ribs 50. The through-holes 24 are formed in the bases of the grooves 48. Each rib 50 is shaped similarly to the ribs 36, e.g., each rib 50 includes an outer peripheral surface 52 which is smooth and which extends circumferentially continuously and also longitudinally. The diameter of the outer surface 52 is equal to that of the outer surfaces 38 of the ribs 36 on the rear portion 30. It will thus be appreciated that the outer surfaces 52, 38 define the outer peripheries of the front and rear portions 22, 30, respectively, as well as the maximum outer diameter of the anchor.

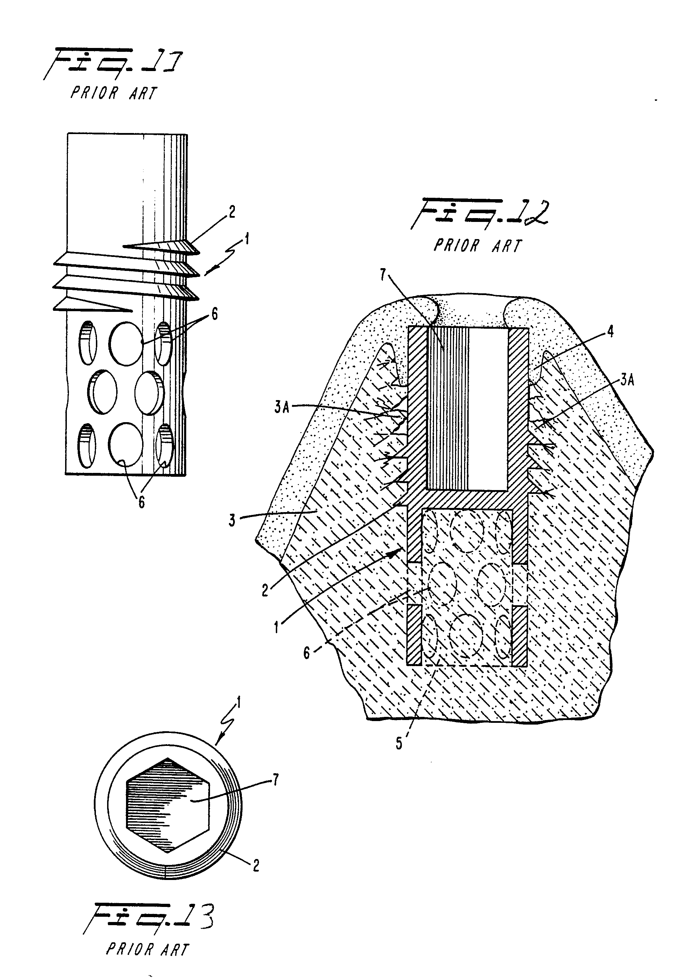

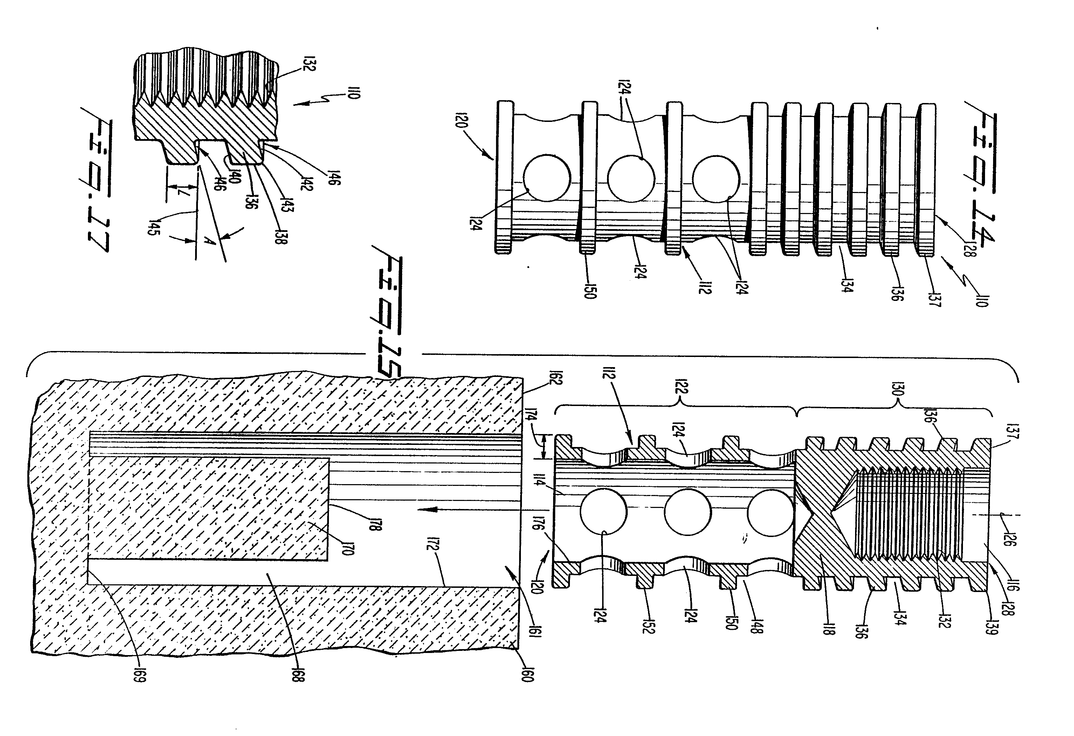

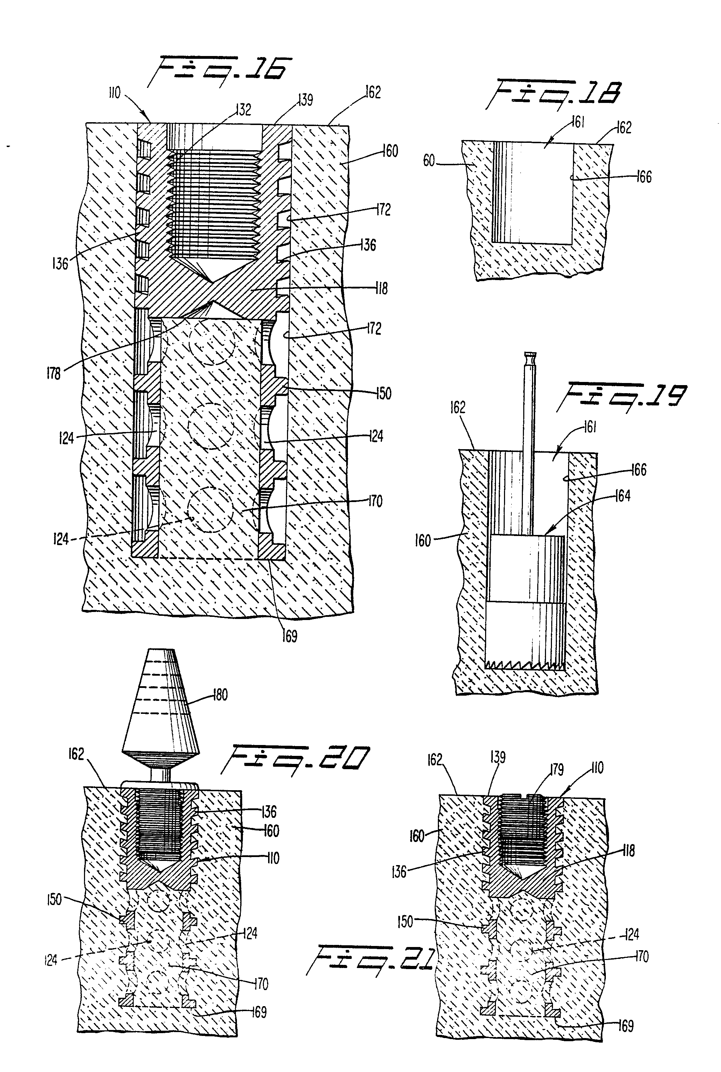

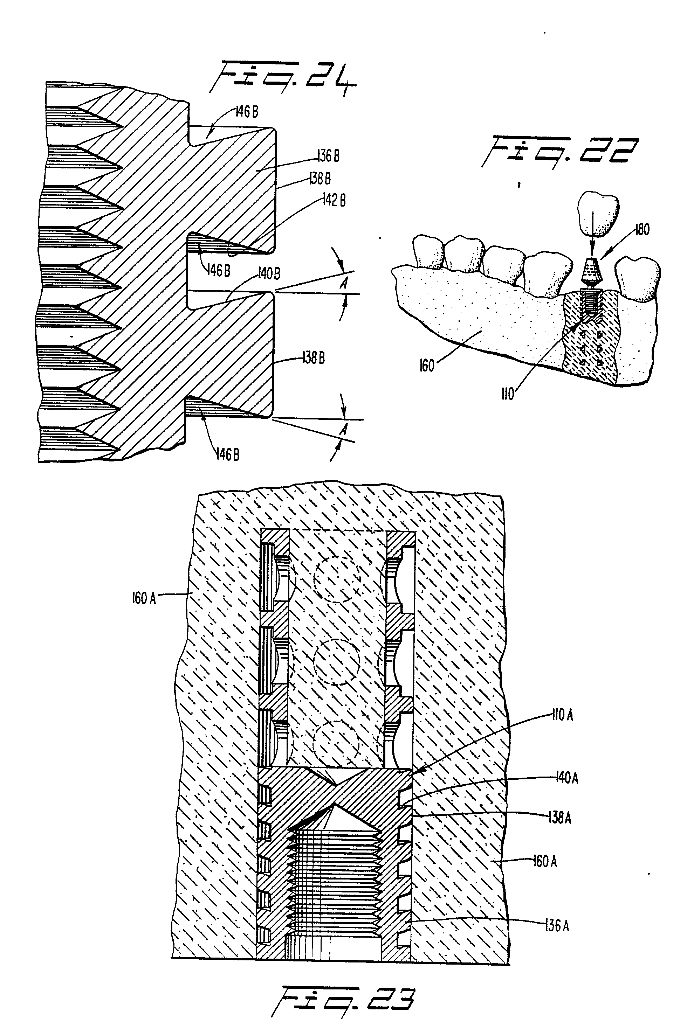

The anchor is installed in accordance with the following procedure. The gum overlying the area of the jaw bone 60 of either the upper or lower jaw in which the anchor is to be affixed is cut and folded back to expose the bone surface 62. A circular cylindrical bore 61 is then formed in the bone. This can be performed in stages wherein a trephine drill 64 cuts a circular kerf in the bone to form an expendable core of bone. That expendable core of bone is removed to leave a cylindrical hole 66 as depicted in FIG. 6. The drill 64 is then further advanced into the bone (FIG. 7) to create an additional kerf 68 (FIG. 2) which, in turn, defines a circular cylindrical permanent core 70. The bore now contains a circular cylindrical inner wall 72 of uniform diameter from the bone surface 62 to the base of the kerf 68. The drill size is selected such that the diameter of the wall 72 corresponds to the diameter of the outer surfaces 38, 52 of the ribs 36, 50, and such that the radial thickness of the kerf 68 corresponds to the radial thickness 74 (FIG. 2) of the front portion 22, i.e., the distance from an inner wall 76 of the front cavity to the outer surfaces 52 of the ribs 50. Thus, the outer diameter of the permanent core 70 corresponds to the diameter of the inner wall 76 of the front cavity.

In addition, the longitudinal distance from the end face 78 of the core 70 to the jaw bone surface 62 is equal to the sum of the longitudinal thickness of the divider wall 18 and the longitudinal dimension of the rear cavity 16 (see FIG 3). Furthermore, the longitudinal dimension of the kerf 68 is at least equal to that of the front cavity 14.

The anchor is installed by being pushed, front end first, into the bore 61. Due to the above-described dimensional relationships, the outer periphery of the body, as defined by the outer surfaces 38, 52 of the ribs, will slide frictionally along the inner wall 72 of the bore, and the front cavity 14 will telescopingly receive the core 70 as the inner wall 76 of the front cavity slides frictionally along the outer peripheral surface of the core.

Therefore, once pushed-in, the anchor will be snugly held within the bore (FIG. 3). This is achieved without cracking or traumatizing the bone and without breaking the core 70. Hence, no undue delays in the initiation of bone regrowth will occur. Furthermore, since the circumferentially uninterrupted outer surfaces 38, 52 press against the wall 72 of the bore or at least are in extremely close proximity to the wall 72, the tendency for blood to gravitate along the wall 72 will be resisted, thereby promoting the formation of blood clots in the immediate vicinity of the bone tissue. The formation of blood clots leads to the development of the necessary cells for bone formation, such as fibroblasts and osteoblasts. As a result, the regeneration and regrowth of bone tissue is promoted. Thus, it is assured that such bone regrowth will not only occur, but will occur rapidly without undue delay. Accordingly, the healing process will be accelerated.

As noted earlier herein, the lack of blood available to bone tissue may not only retard bone regrowth, but may even result in an absence of bone regrowth in the blood-deprived areas. One such area, for example depicted at 4 in FIG. 12, is situated at the mouth of a bore formed in the lower jaw bone since blood will flow from that area gravitationally. However, the presence of blood clots adjacent the rear portion of the anchor eliminates the possibility that the bone tissue near the mouth of the bore will be deprived of blood. Furthermore, since the bone is not fractured by the anchor (in contrast to anchors employing self-tapping threads), the anchor will be in contact with healthy bone and thus will be capable of resisting dislodgement of the anchor. Also, no spaces will be created into which soft tissue may grow. The rearwardmost rib 36A is situated at the rearmost end of the anchor and aids in closing-off the mouth of the bore.

Very quickly, then, the bone tissue located externally around the anchor and internally within in the front cavity 14 will grow toward the anchor and become adhered thereto (i.e., adherence occurs since the anchor is formed of a biocompatible material). Bone tissue will also grow into the grooves 34, 48 between the ribs 36, 50 and through the through-holes 24 to strongly resist dislodgement of the anchor.

At the time when the anchor is pushed into the bore 61, the rear cavity preferably contains a threaded-in healing screw 78A (also formed of the afore-described biocompatible material) to prevent the entry of foreign matter into the rear cavity (see FIG. 5). After the healing process has progressed sufficiently, the retaining pin can be removed and replaced by a threaded insert 80 (depicted in FIG 4) to which a dental prosthesis (not shown) may be attached. Thus, the anchor and insert 80 are both installed without the use of cement.

Since there is no need to screw-in the anchor via self-tapping screw threads, there is no need to employ a wrench. Hence, the anchor can be easily installed in all areas of the mouth. Also, the anchor can be inserted into any space which is large enough to receive a single tooth, i.e., the anchor is not restricted to use only in spaces large enough to accommodate the head of a wrench.

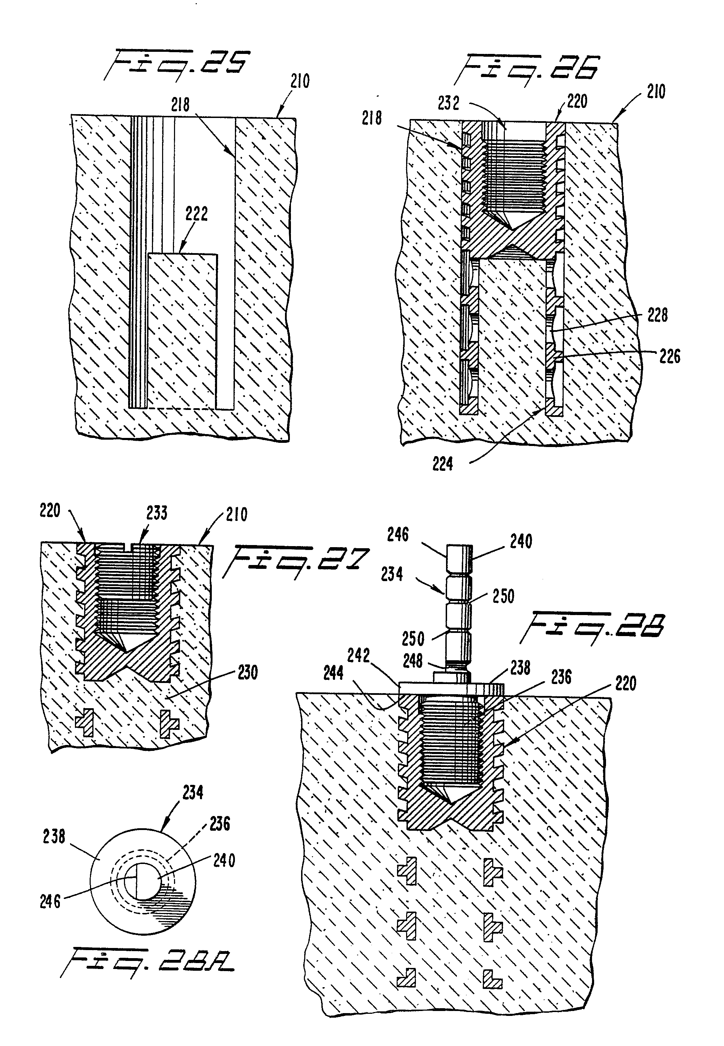

Another preferred embodiment of the invention depicted in FIGS. 8 to 10, involves an anchor 10A which is similar in construction and materials to that disclosed in connection with FIGS. 1 to 3 except that the outer periphery of the body 12A is defined by a smooth cylindrical surface 99 rather than by alternating ribs and grooves. The anchor 10A is installed in the manner disclosed earlier in connection with FIGS. 2, 6 and 7. It will be appreciated that whereas the earlier described anchor 10 involves only a limited number of circumferentially continuous regions (defined by the outer surfaces 38, 52) which frictionally engage the inner wall 72 of the bone, the anchor 10A contains an infinite number of such circumferentially continuous regions which frictionally engage the wall 72. Thus, the entire outer periphery 99 of the anchor 12A will be available for frictional engagement with the wall 72 of the bone to resist blood flow and thereby promote the clotting of blood. Accordingly, the advantages described earlier herein with reference to the anchor 10 of FIGS. 1-3 are applicable as well to the anchor 10A of FIGS. 8 to 10.

## Anchor Having Reservoirs

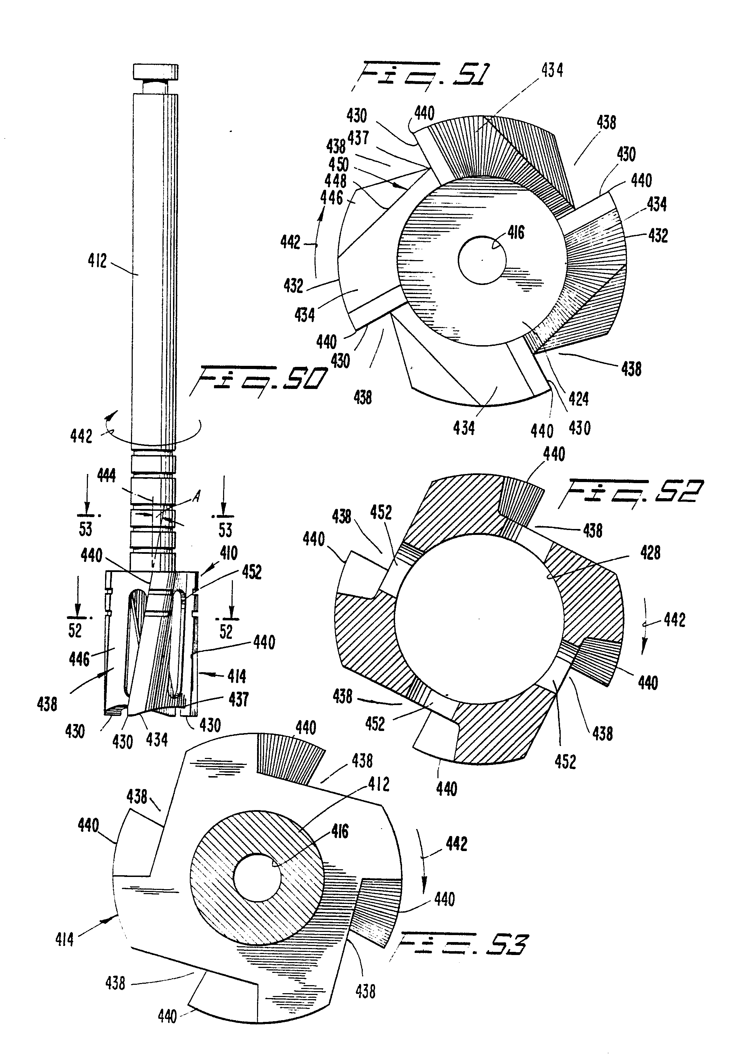

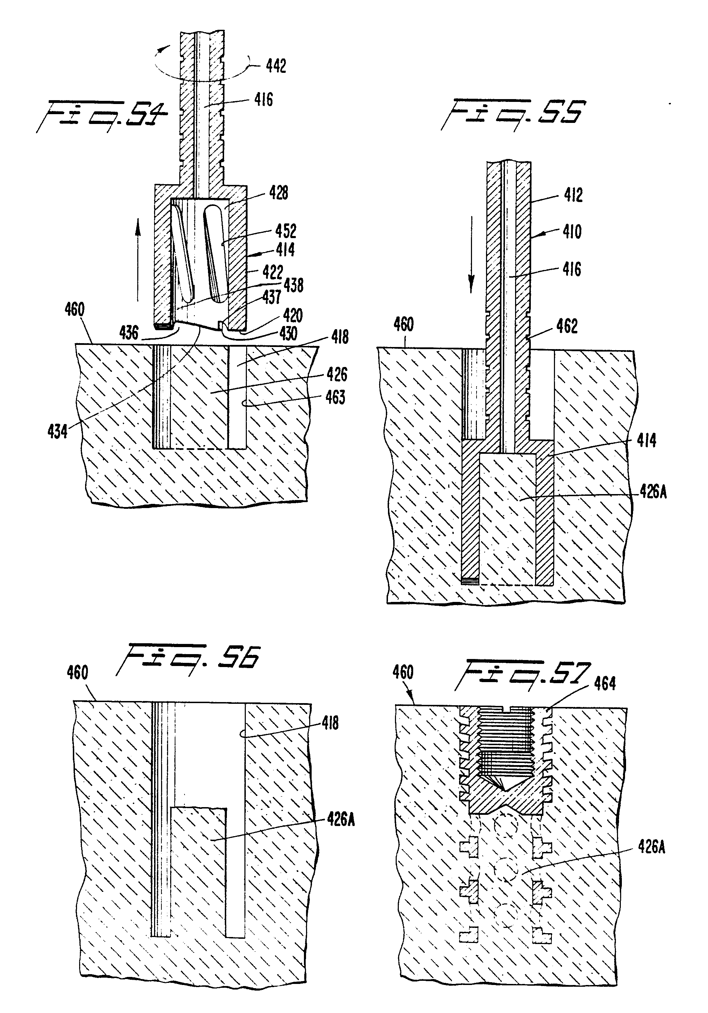

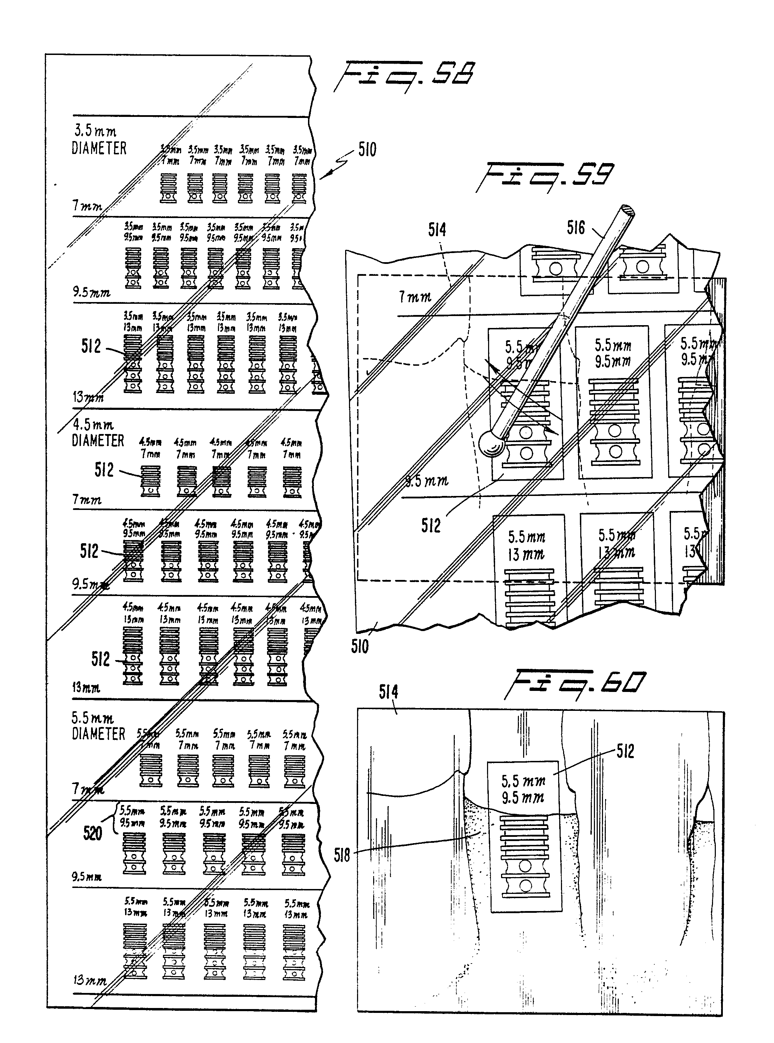

A dental anchor 110 according to the present invention, depicted in FIGS. 14-23, is suitable for use in lower jaw bones. The anchor comprises a generally cylindrical body 112 formed of a biocompatible material such as, for example, titanium such as commercially pure titanium or a titanium alloy such as Ti 6AL 4V, or other equivalent materials as will be apparent to those skilled in the art. The body defines front and rear circular cylindrical cavities 114, 116 which are separated by a divider wall 118 (FIG. 16). The front cavity 114 is open toward a front end 120 of the body 112 and is surrounded by a front portion 122 of the body. That front portion 122 is apertured by means of a plurality of through-holes 124. The through-holes are arranged in circumferential rows, each row being spaced apart in a direction parallel to the longitudinal axis 126 of the body.

The rear cavity 116 opens toward a rear end 128 of the body 112 and is surrounded by a rear portion 130 of the body. That rear portion 130 is non-apertured, i.e., it does not afford any appreciable external communication for the rear cavity except via the rear opening 128. The rear cavity includes an internal wall which contains female screw threads 132.

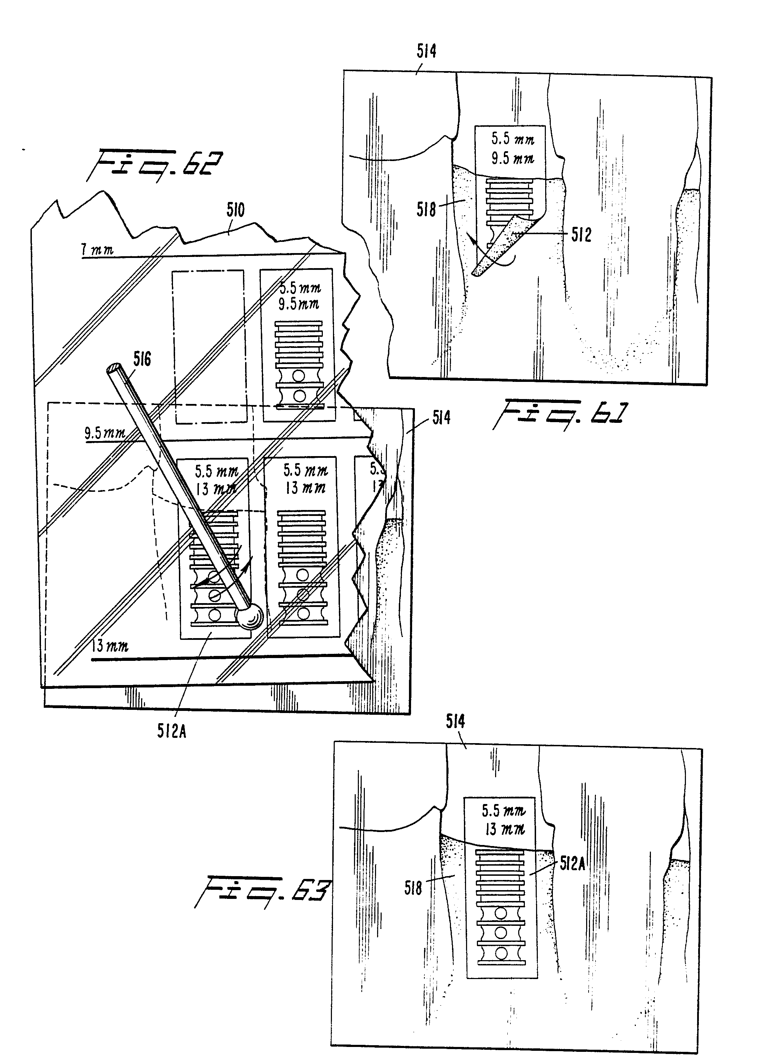

The exterior of the rear portion 130 comprises a plurality of longitudinally alternating grooves 134 and ribs 136. The ribs 136 are circular and extend circumferentially continuously around the body 112. Each rib includes an outer peripheral surface 138 (FIG. 17) which extends circumferentially continuously. That is, the surface 138 is circular as opposed to being helical as in the case of screw threads. Also, the surface 138 has a dimension L in the longitudinal direction. Front and rear edges of the surface 138 are slightly radiused. The outer surfaces 138 are of equal outer diameter and define the outer periphery of the rear portion 130.

Each rib includes front-facing and rear-facing surfaces 140, 142 which are circumferentially continuous. The rear surface 142 is recessed, e.g., preferably offset forwardly relative to a rear edge 143 of the rib outer periphery 138 (which rear edge 143 defines the outer circumference of the surface 142) to define an annular reservoir 146 adapted to store a quantity of blood. Accordingly, a supply of blood will be made continuously available to the bone tissue situated adjacent the reservoirs. The presence of the blood adjacent the bone tissue will lead to the formation of organized blood clots which, in turn, will lead to the development of the necessary cells for bone formation such as fibroblasts and osteoblasts to promote the rapid formation and growth of new bone tissue.

Preferably, the recessing of the rear surface 142 is created by inclining the rear surface 142 forwardly obliquely from the rear edge 143 of the outer periphery 138 of the rib. Thus, the radially innermost edge of the rear surface 142 is disposed forwardly of the edge 143. The rear surface 142 is thus inclined at an angle A relative to a plane 145 oriented perpendicularly to the longitudinal axis 126. Preferably, that angle A is from ten to sixty degrees, most preferably about fifteen degrees.

The rib 137 located at the rear end of the implant does not form a reservoir, but rather has a front face 139 which is flush with the rear end of the anchor and extends perpendicularly to the longitudinal axis so as to completely close-off the mouth of the bore and prevent the growth of soft gum tissue into the bore.

The exterior of the front portion 122 of the body 112 includes longitudinally alternating grooves 148 and ribs 150. The through-holes 124 are formed in the bases of the grooves 148. Each rib 150 includes an outer peripheral surface 152, the diameter of which is equal to that of the outer surfaces 138 of the ribs 136 on the rear portion 130. It will thus be appreciated that the outer surfaces 152, 138 define the outer peripheries of the front and rear portions 122, 130, respectively, as well as the maximum outer diameter of the anchor.

The ribs 150 on the front portion 122 may, if desired, include forwardly offset rear surfaces similar to those discussed earlier herein in connection with the ribs 136 of the rear portion 130, so as to define blood-retaining reservoirs. However, ribs of the reservoir-defining type are of greater dimension in the longitudinal direction, and thus would reduce the number of through-holes 124 which could be employed. It is preferable to maximize the number of through-holes since they accommodate the growth of bone tissue through the body to anchor the body in the jaw bone. Furthermore, the need to store blood at the front portion 122 of the body 112 may be less critical than at the rear portion 130 since blood gravitates away from the mouth of a bore formed in the lower jaw bone.

The anchor 110 is installed in accordance with the following procedure. The gum tissue overlying the area of the lower jaw bone 160 in which the anchor is to be affixed is cut and folded back to expose the bone surface 162. A circular cylindrical bore 161 is then formed in the bone. This can be performed in stages in accordance with a conventional technique wherein a trephine drill 164 cuts a circular kerf in the bone to form an expendable core of bone. That expendable core of bone is removed to leave a cylindrical hole 166 as depicted in FIG. 18. The drill 164 is then further advanced into the bone (FIG. 19) to create an additional kerf 168 (FIG. 15) which, in turn, defines a circular cylindrical inner wall 172 of uniform diameter from the bone surface 162 to the base 169 of the kerf 168. The drill size is selected such that the diameter of the wall 172 corresponds to the diameter of the outer surfaces 138, 152 of the ribs 136, 150, and such that the radial thickness of the kerf 168 corresponds to the radial thickness 174 (FIG. 15) of the front portion 122, i.e., the thickness from an inner wall 176 of the front cavity to the outer surfaces 152 of the ribs 150. Thus, the outer diameter of the permanent core 170 corresponds to the diameter of the inner wall 176 of the front cavity 114.

In addition, the longitudinal distance from the end face 178 of the core 170 to the jaw bone surface 162 is equal to the sum of the longitudinal thickness of the divider wall 118 and the longitudinal dimension of the rear cavity 116. Furthermore, the longitudinal dimension of the kerf 168 is at least equal to that of the front cavity 114.

The anchor is installed by being pushed, front end first, into the bore 161, preferably by hand and without the need of a wrench. Due to the above-described dimensional relationships, the outer periphery of the body, as defined by the outer surfaces 138, 152 of the ribs, will slide frictionally along the inner wall 172 of the bore, and the front cavity 114 will telescopingly receive the core 170 as the inner wall 176 of the front cavity slides frictionally along the outer peripheral surface of the core.

Therefore, once pushed-in, the anchor will be snugly held within the bore (FIG. 16). This is achieved without cracking or traumatizing the bone and without breaking the core 170. Hence no undue delays in the initiation of bone regrowth will occur. Furthermore, since the circumferentially uninterrupted outer surfaces 138, 152 engage the wall 172 of the bore or at least are in immediate proximity to the wall 172, the tendency for blood to gravitate along the wall 172 will be resisted, thereby promoting the formation of blood clots. Importantly, blood will be stored within the reservoirs 146 and will begin to clot. The accumulation and retention of blood clots in the immediate vicinity of bone tissue promotes the formation of organized blood clots which, in turn, leads to the development of the necessary cells for bone formation, such as fibroblasts and osteoblasts. As a result, the regeneration and regrowth of the bone tissue is promoted. Thus, it is likely that such bone regrowth will not only occur, but will occur rapidly without undue delay. Accordingly, the healing process will be accelerated.

As noted earlier herein, the lack of blood available to bone tissue may not only retard bone regrowth, but may even result in an absence of bone regrowth in the blood-deprived areas as soft tissue grows into the areas between the bone and anchor. However, the presence of blood reservoirs 146 in the rear portion of the anchor eliminates the possibility that the bone tissue near the mouth of the bore will be deprived of blood, but rather insures that an ample supply of blood and blood clots will be provided. Furthermore, since the bone is not fractured by the anchor (in contrast to anchors employing self-tapping threads), the anchor will be in contact with healthy bone and thus will be capable of resisting dislodgement of the implant. Also, no spaces will be created into which soft tissue may grow. The rearwardmost rib 137 aids in closing-off the mouth of the bore.

Very quickly, then, the bone tissue located externally around the anchor and internally within in the front cavity 114 will grow toward the anchor and become adhered thereto (i.e., adherence occurs since the anchor is formed of a biocompatible material). Bone tissue will also grow into the grooves 134, 138 between the ribs 136, 150 and through the through-holes 124 to strongly resist dislodgement of the anchor.

At the time when the anchor is pushed into the bore 161, the rear cavity 116 preferably contains a threaded healing screw 179 (also formed of the afore-described biocompatible material) to prevent the entry of foreign matter into the rear cavity (see FIG. 21). After the healing process has progressed sufficiently, the healing screw can be removed and eventually replaced by a threaded insert 180 (depicted in FIG. 21) to which a dental prosthesis (not shown) may be installed without the use of cement.

Since there is no need to screw-in the anchor via self-tapping screw threads, there is no need to employ a wrench. Hence, the anchor can be easily installed in back areas of the mouth. Also, the anchor can be inserted into any space which is large enough to receive a single tooth (see FIG. 21), i.e., the anchor is not restricted to use only in spaces large enough to accommodate the head of a wrench.

The anchor 110 can be utilized in both the upper and lower jaw bones, but it is preferably employed in the lower jaw bone since the ribs will form reservoirs to store blood.

An anchor 110A more suitable for use in an upper jaw bone 160A is depicted in FIG. 23. That anchor is similar to the anchor 110 described in connection with FIGS. 14-21 except that the reservoir-forming ribs 136A are inclined in the opposite direction. That is, the front surface 140A is inclined rearwardly from the front end of the outer peripheral surface 138A of the rib. Hence, the reservoirs face toward the front end of the anchor. The angle of inclination of the surface 140A can be the same as that described earlier with reference to reservoirs 146.

Yet another anchor configuration is depicted in FIG. 24, wherein both rib surfaces 140B, 142B are offset longitudinally relative to the rib outer periphery 138B in a direction away from the anchor ends toward which they face. Accordingly, such an anchor defines reservoirs 146B facing forwardly and rearwardly and can be used in either the upper or lower jaw bone.

## Attachment of Prosthesis to Anchor

Now there will be described in connection with FIGURES 25 to 38 a method for attaching a prosthesis to the installed anchor.

Depicted in FIG. 29 is a portion of the dental arch of a lower jaw 210 in a human mouth. Disposed between two healthy teeth 212, 214 is a gap 216 in which a dental prosthesis is to be placed in accordance with the present invention, the steps of which will first be summarized and then described in greater detail.

A hole 218 is drilled in a portion of the jaw bone disposed within the gap 216 as depicted in FIG. 25, and an implant or anchor 220 is inserted into the hole (FIG. 26). A healing screw 233 (FIG. 27) is threaded into a threaded hole 232 of the anchor during the healing period wherein the anchor becomes permanently affixed to the jaw bone tissue. Then the healing screw is removed and replaced by a pin 234 such that a post portion 240 of the pin projects beyond the jaw (FIG 28). A clay impression 252 of the dental arch (including the post) is taken (FIGS. 29 and 30), whereafter the pin 234 is unscrewed from the anchor 220. A transgingival screw (not shown) is screwed into the anchor hole 232 to prevent gum tissue from growing into that hole while a prosthesis is being prepared. The post 240 of the pin is inserted into the corresponding hole 256 of the clay impression, and a protective sheath 260 is threaded onto the exposed threaded stem 236 of the post (FIG. 31). Then, a dental model 258 is formed from the impression (FIGS 32 and 33), with the sheath 260 becoming embedded therein.

The post 240 is then cut to achieve a desired degree of parallelism with the adjacent teeth and occlusion with the opposing teeth (FIG. 33). A fastener assembly 272 is attached to the post (FIG. 33), and dental wax 270 is applied to the post 240 to form a body shaped and oriented compatibly with the shape and orientation of the adjacent teeth 212', 214' (FIG. 34). In so doing, the sleeve 274 of the fastener assembly 272 becomes embedded in the wax 270.

The unit 277 comprised of the pin 234, the wax body 270, and the fastener assembly 272, is then removed from the dental model and subjected to a conventional investment-type casting operation (FIG. 34A) wherein the wax is melted and replaced by a permanent material, such as gold, in which the sleeve 274 becomes encased (Fig. 35). A layer of release material 294 is applied to the gold body 290 and a wax layer 296 is applied thereover which is shaped in conformance with the desired overlay shape. In a subsequent investment-type casting operation, the wax is replaced by a permanent material such as gold which forms an overlay 292 that can be removed from the gold body upon removal of the screw 276.

A more detailed explanation of the invention will now be provided. Briefly, the hole 218 is drilled in the jaw bone by means of a trephine drill which leaves a cylindrical center core of bone 222. The anchor 220 is pushed longitudinally into the hole such that the core 222 enters a front recess 224 of the anchor (FIG. 26). The anchor side wall 226 includes through-holes 228 through which bone tissue 230 grows (FIG. 27). The anchor thus becomes affixed within the jaw absent the use of cement. A rear end of the anchor 220 includes a threaded hole 232 which opens into the patient's mouth. That hole is adapted to receive a healing screw 233 (FIG. 27) which is inserted into the hole 232 prior to the installation of the anchor into the hole 218 and remains in the hole 232 during the healing process. Thereafter, the screw 233 is replaced by the pin 234.

The stem 236 of the pin 234 projects from one side of a circular flange 238, and the post 240 projects from the other side of the flange 238. The stem and post are both of smaller width (cross-section) than the flange as is evident from FIG. 31. The outer diameter of the flange corresponds to the outer diameter of the anchor. Hence, the outer edge 242 of the flange will lie flush with the outer edge 244 of the anchor 220 to avoid the creation of a space between those members into which food and bacteria could enter. The pin is preferably formed of a biocompatible material such as a titanium alloy or commercially pure titanium.

The post 240 is of non-symmetrical cross-sectional shape (FIG. 28A) due to a flat portion 246 of the post which terminates just above the flange 238. A circumferential groove 248 is formed in the post at a slight distance above the flange 238 (FIG. 28). The outer periphery of the post is of longitudinally irregular shape due to the presence of that groove 248 plus a series of additional circumferential grooves 250, which are spaced apart in the longitudinal direction of the post.

The stem 236 can be screwed into the anchor by hand, or by a tool, if desired. Once the pin 234 has been tightly threaded in place, an impression 254 of the dental arch (including the post 240) is made in a conventional manner such as by pressing a piece of clay 252 over the dental arch (FIGS. 29, 30). The impression 254, which includes a cavity 256 of non-circular cross-section formed by the post 240, serves as a mold to create a plaster dental model 258 (FIG. 33) of the arch in a conventional manner. Prior to forming the dental model, the pin 234 is unscrewed from the anchor 220 and replaced with a transgingival screw (not shown) which is screwed into the anchor hole 232. The transgingival screw includes a head which projects beyond the gum line to prevent the gum from growing over the anchor. After being removed from the anchor, the post 240 is inserted into the cavity 256 as depicted in FIG 31. Due to the non-symmetrical cross-sectional shape of the cavity 256, the position of the post 240, once inserted into the cavity 256, will correspond precisely to the position of the post in the jaw when the impression was made. Hence, when the dental model is made, the orientation of the post relative to the adjacent teeth 212', 214' of the model 258 will correspond precisely to the orientation of the post 240 relative to the actual teeth 212, 214 of the patient.

It is desirable to protect the screw thread of the stem 236 during the formation of the dental model. Accordingly, a sheath 260 (FIG. 31) is positioned over the stem 236 before the model is formed. The sheath 260 is of generally cylindrical configuration and includes a threaded recess at one end and a cut-out 262 or step at the opposite end. A circumferential groove 264 is provided in the outer surface of the sheath 260. The threaded recess of the sheath is screwed onto the threaded stem 236 of the pin 234, and then the dental model 258 is formed by pouring plaster or dental stone 266 into the clay impression in a conventional manner (FIG. 32). Accordingly, the sheath 260 becomes embedded in the dental model (FIG. 33). Rotation of the sheath 260 within the dental model is prevented due to the non-circular cross-section defined by the step 262, and longitudinal dislodgement of the sheath 260 from the model 258 is prevented due to the longitudinally irregular periphery defined by the groove 264 in the sheath.

As a result, the operator possesses an identical model 258 of the patient's dental arch (FIG. 33), with the pin 234 removably threaded in the model. The operator is then able to form the post 240 with a shape that is compatible in size and orientation to the adjacent teeth 212', 214', and with a height that is compatible with the jaw-to-jaw height. This forming operation is accomplished by suitably cutting the post 240 and applying to the post a heat-meltable substance capable of being worked and shaped, such as dimensionally stable dental wax 270. The cutting and waxing of the post is performed while the pin 234 is mounted in the dental model 258. As a result, the shaping of the pin can be achieved without discomfort to the patient and with superior accuracy, since the shaping can be performed with precision tools and guides which cannot be employed within the confines of a patient's mouth. It is important to note that since the cavity 256 defined in the clay 252 by the post 240 was of non-symmetrical cross-sectional shape, the post could be inserted back into that cavity in only one possible position, i.e., a position corresponding to its position within the anchor 220. Thus, the position of the post relative to the adjacent teeth 212', 214' within the model 258 will correspond precisely to the position of the post 240 relative to the teeth 212, 214 when the pin is eventually screwed into the anchor 220. Therefore, the parallelism achieved by the post and wax body relative to the model teeth 212', 214' will be carried forward relative to the teeth 212, 214 when the pin is eventually screwed back into the anchor 220.

Prior to the waxing step, the fastener assembly 272 is attached to the post 240. The fastener assembly comprises the threaded sleeve 274 and the screw 276, both formed of a suitably heat-resistant alloy. The sleeve 274 contains internal threads for threadedly receiving the screw 276. The head 278 of the screw is adhered to a piece of sticky wax 280 carried at the end of a manually held rod 282 to enable the fastener assembly 272 to be manually located at a desired location relative to the post 240. Then the sleeve 274 is adhered to the post 240 by a bit of heat-meltable wax 284 which quickly hardens to enable the piece of sticky wax 280 to be removed, leaving the fastener assembly 272 adhered to the post 240 (FIG. 33). The fastener assembly 272 can be oriented parallel to the post, or at an angle relative thereto; the assembly 272 can be disposed vertically or horizontally if desired.

The dimensionally stable dental wax 270 is then applied to the post to create a shaped body (FIG. 34) which is compatible in shape and orientation to the adjacent teeth. The wax is applied so as to encase the sleeve 274 while leaving the head 278 of the screw 276 exposed. The flange 238 of the pin 234 serves as a shelf upon which the wax 270 is applied.

The preliminary unit 277 comprising the pin 234, the shaped wax body 270, and the fastener assembly 272 is unscrewed from the dental model, i.e., unscrewed from the sheath 260. This can be performed by a pliers-like tool which engages the outer edge 242 of the flange to avoid damaging or deforming the wax body 270. The unit is then placed in a conventional investment-type dental casting material and machine which replaces the wax 270 with a permanent material such as gold, porcelain, acrylic, etc. The nature of that casting process is well known in the art and can be summarized as follows. The wax body is attached to a sprue 286 formed of a meltable material such as plastic. The entire unit and sprue are encased in a conventional pliable investing material 288 (FIG. 34A) which is pressed against the outer periphery of the unit 277. A conventional investment material can be used. Thereafter, the invested unit 277 is subjected to sufficient heat to melt the wax 270, 284 and the sprue 286. When this melting occurs, the fastener assembly 272 is held in place relative to the post 240 since the head 278 of the screw is embedded in the investment material (FIG. 34A). Then, under the influence of centrifugal force, the melt is discharged and replaced by a liquified permanent material, such as melted gold, which occupies the void created by the discharged melt. Upon hardening of the gold, the unit 277' appears identical to its previous appearance except that the wax body 270 has been replaced by gold 290 (FIG. 35). The unit 277' thus forms a secondary unit for receiving the overlay or crown. The sleeve 274 may be provided with projections or grooves (not shown) to enhance its securement within the gold body 290.

Next to be formed is the overlay 292 which is to be removably attachable to the secondary unit 277'. The overlay 292 is formed by applying a conventional release or parting material 294 to the outer periphery of the gold body 290 and the head 278 of the screw 276, and then forming a layer 296 of dimensionally stable wax over the parting material. These steps are performed after the secondary unit 277' has been screwed back into the sheath 260 of the dental model 258. The wax layer 296 is shaped in accordance with the desired shape of the overlay 292. The wax layer 296 and screw 276 are removed from the body 290, and the screw is reinserted into the wax layer 296. The assembly of the layer 296 and screw 276 is inserted into an investment-type casting material and machine, whereupon the wax layer 296 is replaced with a permanent material such as gold, porcelain or acrylic for example, by means of an investment-type casting operation, to form the overlay 292. Due to the presence of the parting material, the overlay does not adhere to the head of the screw. The overlay can be placed upon the body 290 and attached thereto by the screw 276. Thus, if the overlay 292 becomes damaged or worn, it can be easily replaced. If the overlay 292 is formed of gold, a porcelain coating can be formed thereon by a conventional baking process. The insert comprised of the unit 277' is attached to the anchor 220 by screwing the stem 236 into the threaded hole 232 of the anchor 220. Then, the overlay 296 is attached by the screw 276. The prosthesis assumes the same degree of parallelism in the patient's mouth as was achieved in the dental model since, upon screwing the pin in place, the pin is rotated to the same rotational position as when screwed into the model. The flange 238 abuts flush against the end of the anchor. Since the flange is formed of a biocompatible material, the gum tissue will be able to grow into engagement with the edge 242 of the flange and thereby hide the flange from view. The overlay may comprise a single tooth, or a bridge 292A (FIG. 38) comprised of a plurality of teeth. If a bridge 292A is to be formed, then in addition to the wax layer 296 applied to the parting material 294, one or more additional wax bodies (not shown) are formed which are interconnected at their sides and one of which being connected to the wax layer 296. That entire assembly is then cast to form a bridge 292A comprising the portion 292 attached to the secondary unit, and one or more adjacent teeth 299 attached to the portion 292.

In accordance with the present invention there is provided a novel method of forming a dental prosthesis. The use of a base member in the form of a pin comprised of a threaded stem, a flange, and a post provides significant advantages. The post is capable of being cut and shaped while disposed within a dental model and provides a rigid structure onto which the dental wax can be applied. Thus, the task of forming a prosthesis which conforms to the shape and orientation of the adjacent teeth and the jaw-to-jaw height is greatly facilitated. The flange itself forms a shelf upon which the dental wax can be supported. The threaded stem enables the pin to be inserted into the anchor without the use of cement. In fact, the present system completely avoids the use of cement, thereby eliminating the risk for biodegradation to occur which would create voids into which food and bacteria could enter.

The present invention makes it simple to achieve precise parallelism between the prosthetic tooth (or teeth) and the adjacent teeth in all directions. This parallelism can be made with precision since the post 240 is shaped outside of the mouth and, thus, shaping tools and guides can be employed which could not be utilized within the confines of the patient's mouth. Even though the shaping of the pin is achieved outside of the mouth, the pin is shaped while oriented in the same position relative to the adjacent teeth as when the pin is eventually replaced into the anchor. This is due to the non-symmetrical cross-sectional shape of the post which forms a corresponding cavity in the clay impression material, thereby assuring that precise registration of the pin occurs in the clay impression and plaster model. Since the height of the pin can be shortened, precise jaw-to-jaw height can be achieved. Therefore, the teeth can be made to occlude (bite) properly.

The post itself is shaped to achieve a positive and reliable attachment to the gold body. That is, relative movement between the gold body and post is prevented by the non-circular cross-section and irregular longitudinal profile of the post.

The flange is circular and engages a circular end of the anchor. The flange is of the same diameter as that circular end of the anchor to avoid the presence of voids into which food and bacteria can enter. Also, the prosthesis will present a more pleasing appearance. The absence of gaps between the prosthesis and gums prevents the occurrence of problems with the patient's phonetics.

The incorporation of the fastener assembly within the prosthesis is achieved simultaneously with a casting step; no subsequent cementing or soldering is required. This is made possible by maintaining the screw in position by means of the investment material during the initial casting step to maintain the sleeve in a fixed position even after the wax has been melted. Furthermore, the pin is waxed-up around the sleeve, rather than pushing the sleeve into the wax. Thus, the wax body will not be deformed.

Since the crown portion is removable, due to the presence of the removable screw, the prosthesis can be easily repaired when damaged or worn.

By forming the pin of titanium, a high degree of biocompatibility with the gum tissue exists, promoting the chances for a seal or hemi-dessmissomal adaptation of gum tissue to be created above the anchor.

It will be appreciated that the fastener assembly can be disposed in virtually any location and orientation so as to be adapted to all types of repair and restorations from an aesthetics standpoint.

Attention is also directed to the fact that the present invention makes it possible to form the base and overlay outside of the patient's mouth, thereby minimizing discomfort to the patient.

Drilling of Bore in Bone Tissue

There will now be discussed in conjunction with FIGURES 39-49 a technique for drilling a bore in a jaw bone for receiving a dental anchor.

Depicted in FIGURE 39 is a jaw bone of a patient in which a dental anchor is to be installed. In a first stage of the operation, a pilot hole 312 drilled to a predetermined depth D' to enable a guide bushing 313 to be installed. The bushing, depicted in FIG. 40, includes first and second cylindrical sections 314, 316, one section 314 being of smaller diameter than the other 316. The diameter of the pilot hole 312 is sufficiently large to receive the small section 314 of the bushing and sufficiently deep to enable an end 318 of the large section 316 to abut the bone tissue 310.

The diameter of the large section 316 corresponds to the inner diameter of a hollow drill head 320 of a trephine drill 322 to enable that hollow head of the drill to be inserted telescopingly over the large section 316 of the bushing (FIG. 41). Thus, the drill is constrained against sideways (radial) movement. The trephine drill 322 includes a shank 324 mounted in a chuck 325 of a hand-held motor housing 326 and is advanced into the jaw bone while the large section 316 of the bushing prevents sideways movement of the drill.

In practice, the drill is advanced longitudinally to a desired depth D", which depth D" is monitored by the operator who must view markings provided along the side of the drill 322. That practice is time-consuming and can be imprecise since the possibility exists for the operator to exceed the desired depth. The depth reached during this stage of drilling determines the depth of the top face of the bone core to be formed in the final bore.

In accordance with the present invention, however, the longitudinal length L of the guide section 16 corresponds to the desired depth D" to be attained during this drilling stage. Thus, when an inner shoulder 328 within the drill head 320 abuts against a stop surface 330 of the guide section 316, the drilling advancement ceases at the current drilling depth D" (Fig. 42).

At this point, the drill 322 and bushing 313 are removed from the initial bore 323 which bore contains an unwanted center core 332 of bone tissue. Accordingly, a hand-held tool 334 is inserted into the bore (FIG. 43) to apply sideways pressure to the core 332 which breaks off and is removed (FIG. 44).

In the next drilling stage, the depth of the initial bore 323 is extended. Importantly, the final depth of the bore must not exceed a predetermined depth $D'''$ in order to absolutely prevent any chance that a nerve will be contacted and damaged.

In accordance with the present invention, a stop member 340 is loosely situated on the drill shank 324 atop the drill head 320 for free longitudinal sliding movement between a top surface 342 of the drill head and an end face 344 of the motor housing. The stop member can be installed before or after the drilling of the initial bore 323. The stop has an outer diameter d larger than that of the drill head 320. The length H of the stop 340 is chosen to assure that the longitudinal advancement of the drill terminates when the desired bore depth $D'''$ has been reached. This is achieved by matching the stop to the drill itself. Since the distance X from the front end 346 of the drill to the end face 344 of the chuck is constant and known, the presence of the stop 340 will reduce the extent of travel of the drill by an amount equal to the length H of the stop. In other words, X minus H equals the desired predetermined depth $D'''$ of the bore 323. Hence, a set of stops of different lengths can be prepared to create a variety of drilling depths $D'''$. As the drilling progresses, the stop 340 will eventually become sandwiched between the end face 344 of the drill chuck and the jaw bone 310 (FIG. 47) to prevent further advancement of the drill 322, whereupon the drill is removed (FIG. 48). A core 350 of bone tissue remains in the final bore 345.

The dental anchor 352 is then inserted into the final bore as depicted in FIG. 49 wherein the core 350 enters a hollow front end of the anchor. Eventually, the bone tissue grows against the anchor 352 to permanently retain the anchor 352 in the jaw bone 310.

It will be appreciated that the stop member 340 of the present invention absolutely assures that the predetermined drilling depth $D'''$ will not be exceeded. This is achieved without the need for an operator to monitor any difficult-to-see markings on the drill. Nor is there any need for a set screw to secure the stop member nor the concern that such a set screw could become inadvertently loosened.

The guide bushing 313 assures that the end surface 356 of the core 350 in the final bore 345 will be at the proper depth. Again, this is achieved without any need for the operator to monitor depth markings on the side of the drill.

## Drill Structure

There will now be described in connection with FIGURES 50-57, a trephine drill according to the present invention for drilling a bore in the jaw bone.

·A dental trephine drill 410 according to the present invention includes a mounting shank 412 and a cutting head 414 disposed at a longitudinal front end of the shank. A longitudinal fluid passage 416 extends through the shank for conducting flushing fluid such as a saline solution or sterile water for flushing cuttings from a bore 418 cut by the drill.

The head 414 is hollow and includes a front end face 420 and a side face 422 extending longitudinally rearwardly from the end face 420. The end face surrounds a center hole 424 which is adapted to receive a core 426 formed during the cutting of a bore (FIG. 54). The fluid passage 416 communicates with the interior 428 of the head 414 and thus with the hole 424.

Formed in the end face 420 is a plurality of first cutting edges 430 extending radially outwardly from the hole 424 to an outer periphery 432 of the front face 420.

Surface portions 434 of the front face 420 disposed circumferentially behind the end cutting edges 430 extend longitudinally rearwardly from the end cutting edges (see FIGS. 50 and 54) to define radial openings 436 which accommodate a flow of drilling fluid. A circumferentially rear end of each such surface portion 434 terminates adjacent the next end cutting edge 430 at a location 437 spaced slightly longitudinally rearwardly and radially inwardly of such end cutting edge to define a relief which accommodates the flow of drilling fluid.

Formed in the cylindrical side face 422 are a plurality of longitudinally oriented grooves 438 which extend longitudinally rearwardly from the end face 420. Each groove 438 includes a longitudinally extending edge 440 which defines a side cutting edge. That side cutting edge 440 constitutes a trailing edge of the respective groove 438 with reference to the direction of rotation 442 of the drill.

The grooves 438 and associated side cutting edges 440 preferably extend at an acute angle A of from 5 to 15° (preferably about 10°) relative to the longitudinal axis of rotation 444 (i.e., the edges 440 are raked rearwardly). All points on the side cutting edge 440 are spaced radially equidistantly from the axis 444, so that the side edges 440 cut a cylindrical bore.

The end cutting edges 430 intersect and extend radially inwardly from longitudinal front ends of respective side cutting edges 440. As can be seen in FIG. 51, the floor 446 of each groove 438 intersects a surface 434 of the end face along a line 448 which extends from the outer periphery 434 of the end face to the location 437, which location 437 is situated radially inwardly from the outer periphery 434. It will be appreciated that each line 448 constitutes an outer edge of a respective surface portion 434 of the end face 420.

The above-described configuration of the grooves 438 results in the formation of longitudinal passages or reliefs 450 (FIG. 51) which communicate the grooves with the end face 420 to accommodate the flow of drilling fluid therebetween.

The floor 446 is not planar, but rather is slightly curved so as to be visible in a front end view (FIG. 51).

Extending through the floor 446 of each groove is an aperture 452 which communicates each groove with the interior 428 of the head 414. Thus, drilling fluid can be conducted radially outwardly from that interior 428 to the grooves 438 in order to cool the drill and bone and to flush cuttings from the grooves 438, as well as prevent fluid pressure build-ups. The apertures 452 are arranged to extend in a longitudinal direction. That is, the apertures may be of longitudinally elongate slot shape as depicted, or each aperture could comprise a series of small longitudinally spaced holes. Such a longitudinally extending configuration ensures that drilling fluid can enter the grooves through the apertures, even as a bone core is being progressively received in the cylindrical interior of the head 414. Also, such fluid cools the drill head and the bone tissue of the bore wall.

IN OPERATION, the drill head 414 is advanced into a jaw bone 460 of a patient. The end cutting edges 430 cut an annular kerf or bore 418 in the bone, leaving a center core of bone 426. Pressurized drilling fluid (e.g., saline solution or sterile water) flows through the front hole 424 of the drill head to clean and cool the cutting edges and flush cuttings from the bore. The outer wall 463 of the bore is cut by the side cutting edges 440. Drilling fluid flows radially outwardly through the apertures 452 into the grooves 438 to clean and cool the side edges 440 and flush cuttings from the bore.

Eventually, as the core 426 enters the front hole 424 of the head, the flow of drilling fluid through the hole 424 is seriously restricted. However, the flow of drilling fluid through the apertures 452 prevents excessive hydraulic pressure build-ups within the drill. Also, such fluid cools the drill head and the bone tissue of the bore wall.

Furthermore, since the grooves 438 communicate with the end face 420 and with the end cutting edges 430 thereon, via passages 450, fluid is able to flow longitudinally forwardly to clean and cool the end cutting edges 430 to compensate for a lack of fluid from the hole 424.

Since the apertures 452 extend longitudinally along the grooves 438, the pressurized fluid continues to be discharged therethrough, even as the apertures 452 become progressively blocked by the core 426. The fluid is also able to contact the outer wall of the bone core via the apertures 452 to cool such bone core.

After an initial portion of the bore has been formed (FIG. 54), the core 426 is intentionally broken off and the drill is reinserted into the bore 418 to extend the bore 418 (FIG. 55) and form a new core 426A. The depth of the bore 418 can be monitored by viewing indicator slits 462 formed in the outer periphery of the stem 412 and head 414. Alternatively, a stop can be employed as disclosed earlier herein.

After the bore 418 has been completed, an anchor 464 is pushed into the bore, wherein the core 426A enters a front opening in the anchor. Eventually, bone tissue grows against the anchor and through holes therein to permanently secure the anchor in place.

The drill is preferably formed of corrosive resistant, heat treated, stainless steel No. 420, although other materials may be suitable. Drills of different outer diameter are provided for forming bores of different size to accommodate an appropriate anchor. For example, drills forming bores having a diameter in the range of from 3.5 mm to 5.5 mm are provided.

A dental trephine drill according to the present invention enables a bore and core to be formed quickly and with minimal trauma to the bone and minimal discomfort to the patient. The side cutting edges assure that the outer wall of the bore is cut cleanly without burrs. Thus, it is unnecessary to repeatedly reciprocate the drill within the bore to remove burrs. This results in a shorter drill period and a more accurately dimensioned bore which assures that a proper fit with the anchor will occur.

The presence of apertures in the grooves prevents the creation of hydraulic pressure build-ups which could resist advancement of the drill. Since the apertures extend longitudinally, this pressure relief continues even as the apertures are progressively blocked by the core. The fluid flowing through the apertures cleans and cools the side cutting edges and flushes cuttings from the bore. The fluid is able to travel longitudinally forwardly from the grooves to clean and cool the end cutting edges, since the front ends of the grooves communicate with the area located in front of the end cutting edges. Also, such fluid cools the bone, i.e., the inner wall of the bore and the outer wall of the bone core to minimize the occurrence of thermal damage thereto.

Selection of Proper Anchor

There will now be described in connection with FIGURES 58-63, a method and apparatus for selecting a properly sized anchor and creating a pictorial record of such a selection.

In accordance with the invention, there is utilized a transparent transfer sheet 510, or acetate overlay, on which are provided a plurality of removable pictorial representations of dental anchors 512 of various sizes. Each pictorial representation can be rubbed off the transfer sheet by placing the sheet upon a receiver sheet, with the pictorial representation facing the receiver sheet 514. The opposite side of the transfer sheet overlying the pictorial representation to be transferred is rubbed with a blunt object 516, causing the pictorial representation to be transferred to the receiver sheet.

The receiver sheet 514 comprises an X-ray of a patient's jaw bone. The pictorial representation of an anchor is transferred to the site 518 on the X-ray where the anchor is to be installed. The transfer sheet is then removed to enable the technician to clearly inspect the X-ray to ensure that the selected anchor is of suitable size for the anchor site. The accuracy of this inspection is not adversely affected by the presence of additional indicia which could interfere with a clear viewing of the X-ray, as can occur in the case of conventional transparent overlays. If the technician feels that a different anchor might be more suitable, the technician simply peels off the transferred pictorial representation 512 (FIG. 61) and applies a pictorial representation 512A of different size (FIGS. 62, 63).

A plurality of available anchor sizes are represented on the transfer sheet, with a plurality of pictorial representations of each size being provided so that the sheet can be used repeatedly. Each pictorial representation includes indicia 520 indicative of dimensions of the respective anchor, such as diameter and length.

It will be appreciated that once the desired anchor has been transferred to the X-ray, the X-ray constitutes a permanent pictorial record of the selected anchor, identified by dimensions 520, for future reference and verification.

Furthermore, in order to provide a factor of safety in the anchor selection process, the pictorial representa-tions 512 are of greater magnification than the X-ray 514. Thus, as long as the selected pictorial representation fits within the anchor site 518, it is assured that the corresponding anchor can safely be employed, even if the size of the selected pictorial representation was of borderline acceptability. For example, if the X-ray is at full-scale, the pictorial representations will be magnified, preferably by about 25 percent. On the other hand, if the X-ray is magnified, then the pictorial representations will be of yet greater magnification. For example, if the X-ray is at 25 percent magnification, the pictorial representations would be of about 50 percent magnification.

Although various different aspects of the present invention have been described in connection with preferred embodiments thereof, it will be appreciated by those skilled in the art that additions, modifications, substitutions, and deletions not specifically described may be made without departing from the scope of the invention as defined in the appended claims.

## Claims

1. A dental anchor (10, 10A; 110, 110A) adapted to be installed in a predrilled bore formed in jaw bone tissue for retaining a dental prosthesis, said anchor comprising a generally cylindrical body (12; 12A; 112) defining a longitudinal axis, said body being open at a front end thereof and including a cylindrical front cavity (14; 114) communicating with said open front end for receiving a cylindrical bone core, said front cavity being surrounded by a front portion of said body, said front portion being apertured (24; 124) to accommodate the growth of bone tissue therethrough, said body being open at a non-apertured rear portion thereof and including a rear cavity (16; 116), characterized in that said rear cavity is internally threaded to receive externally threaded prosthesis-attachment means (80; 180), and said front and rear portions of said body including front and rear non-threaded outer peripheries (52 and 38; 99; 152 and 138; 138B), respectively, which are of the same diameter and define a maximum outer diameter of said anchor.

2. A dental anchor according to claim 1, wherein said rear outer periphery includes surface regions (52; 152; 99) which are circumferentially continuous and longitudinally extending to resist the passage of blood longitudinally therepast.

3. A dental anchor according to claim 2, wherein all of said rear outer periphery (99) is of uniform diameter to define an infinite number of said surface regions.

4. A dental anchor according to claim 2, wherein said rear outer periphery of said body includes circumferentially extending grooves (34; 134) disposed in longitudinally alternating relationship with said circumferentially continuous surface regions.

5. A dental anchor according to claim 4, wherein said surface regions comprise the outer surfaces of ribs (36; 136, 136A, 136B) defined by said grooves, there being additional grooves (48; 148) in said front portion disposed in longitudinally alternating relationship with apertures in said front portion.

6. A method utilizing the anchor defined any preceding claim, wherein a closed-ended bore (61; 161) is drilled in a jaw bone such that said bore includes an inner cylindrical wall of uniform diameter extending forwardly from a surface of said bone to a closed front end of said bore, and a cylindrical core (70; 170) extending rearwardly from said closed end, said core including an outer peripheral surface coaxial with said inner wall and spaced radially inwardly therefrom, characterized in that said core and inner wall define an annular kerf (68; 168) having a radial dimension substantially equal to a radial thickness (74; 174) of said front portion of said body, said outer peripheral surface of said core defining a diameter substantially equal to a diameter of said front cavity of said anchor, said anchor being pushed into said bore such that said outer peripheries (52 and 58; 99; 152 and 138; 138B) of said front and rear portions of said body frictionally slide against said inner wall of said bore without penetrating said inner wall, and an inner wall (76; 176) of said front cavity frictionally slides against said outer wall of said core as said core enters said front cavity, the insertion of said anchor being terminated such that said inner wall of said front cavity frictionally engages said outer peripheral surface of said core, and said outer peripheries of said body frictionally engage said inner wall of said bore to resist the travel of blood therepast.

7. A dental anchor (110) adapted to be installed into a predrilled bore formed in jaw bone tissue for retaining a dental prosthesis, said anchor comprising a generally cylindrical body (112) defining a longitudinal axis and including an outer periphery, said body being open at a front longitudinal end thereof and including a front cylindrical cavity (114) communicating with said open front end for receiving a cylindrical bone core, a plurality of through-holes (124) formed in said body and communicating with said front cavity to accommodate the growth of bone tissue therethrough, said body being open at a rear longitudinal end thereof and including a rear cavity (116) communicating with said open rear end for receiving a prosthesis-attachment means, characterized in that a plurality of longitudinally spaced reservoir-defining means (136; 136A, 136B) are formed in said outer periphery, each said reservoir-defining means including a recessed surface (142; 140A,; 142B; 140B) facing generally toward one of said longitudinal ends and arranged to store blood adjacent to surrounding bone tissue.

8. A dental anchor according to claim 7, wherein each of said reservoir-defining means comprises a circumferentially extending rib (136; 136A, 136B) containing said recessed surface.

9. A dental anchor according to claim 8, wherein each said rib is circumferentially continuous.

10. A dental anchor according to claim 7, 8 or 9 wherein at least some of said surfaces (142; 142B) face toward said front end of said anchor.

11. A dental anchor according to claim 10, wherein each of said surfaces is inclined rearwardly obliquely from its outer circumference.

12. A dental anchor according to claim 11, wherein each of said surfaces is inclined at an angle of from 10 to 60 degrees relative to a plane oriented perpendicularly to said longitudinal axis.

13. A dental anchor according to claim 12, wherein said angle is 15 degrees.

14. A dental anchor according to any of claims 7 to 13 at least some of said surfaces (140A, 140B) face toward said rear end of said anchor.

15. A dental anchor according to claim 14, wherein each of said rear facing surfaces is inclined rearwardly obliquely from its outer circumference.

16. A dental anchor according to claim 15, wherein each of said rear facing surfaces is inclined at an angle of from 10 to 60 degrees relative to a plane oriented perpendicularly to said longitudinal axis.

17. A dental anchor according to claim 16, wherein said angle is 15 degrees.

18. A dental anchor according to any of claims 7 to 17 wherein some of said reservoir-defining surfaces (142; 142B) face toward said front end of said anchor, and others of said reservoir-defining surfaces (140A; 140B) face toward said rear end of said anchor.

19. A dental anchor (110; 110A) adapted to be inserted into a predrilled bore formed in jaw bone tissue for retaining a dental prosthesis, said anchor comprising a generally cylindrical body (112) defining a longitudinal axis and including an outer periphery, said body being open at a front end thereof and including a front cylindrical cavity (114) communicating with said open front end for receiving a cylindrical bone core, a plurality of through-holes (124) formed in said body and communicating with said front cavity to accommodate the growth of bone tissue therethrough, said body being open at a rear end thereof and including a rear cavity (116) communicating with said open rear end for receiving prosthesis-attachment means, a plurality of longitudinally spaced, circumferentially extending grooves (134) formed in said outer periphery to define longitudinally spaced, circumferentially disposed ribs (136; 136A, 136B) including circumferentially continuous outer periphery of equal diameter, said ribs each including a front surface (142; 142B) facing toward said front end and a rear surface facing toward said rear end, characterized in that at least one of said front and rear surfaces being offset longitudinally relative to said outer periphery of said rib in a direction away from the respective anchor end toward which said one surface faces to define an annular reservoir adapted to store blood adjacent to surrounding bone tissue.

20. A method of forming a dental prosthesis comprising the steps of:

attaching an internally threaded sleeve (274) to a post (240),

applying a dimensionally stable, heat-meltable material (270) to said post and around said sleeve, and

melting said heat-meltable material and replacing same with a permanent material (277').

21. A method of forming a dental prosthesis and securing same in the jaw bone of a patient, comprising the steps of:

drilling a bore (218) in the patient's jaw bone,

inserting into said bore an anchor (220) formed of a material which is biocompatible with bone tissue, said anchor including an internally threaded opening (232) accessible from within the patient's mouth,

inserting into said threaded opening an externally threaded stem (236) of a pin (234), said pin including a flange (238) having a first side from which said stem projects and a second side from which a post (240) extends in a direction opposite said stem, such that said flange seats upon said anchor and said post projects away from said anchor,

forming an impression (254) of at least a portion of said jaw including said post, such that said post forms a cavity (256) in said impression,

removing said pin from said anchor and inserting said post into said cavity such that said threaded stem extends away from said impression,

pouring a hardenable substance (266) into said impression to form a dental model (258) in which said stem is disposed,

attaching to said post a fastener assembly (272) comprising an internally threaded sleeve (274) and a screw (276) threaded therein,

forming upon said flange and around said post and. sleeve a shaped mass (270) of dimensionally stable, heat-meltable material, and

melting said heat-meltable material and replacing same with a permanent material (290) to form a permanent body (277').

22. A combination of a dental anchor (220) securable within a patient's jaw and a pin (234) upon which a dental prosthesis is to be formed, said pin being mountable within said anchor, said anchor formed of a biocompatible material and configured for installation in a generally cylindrical hole (218) in a patient's jaw bone, an internally threaded opening (232) formed at one end of said anchor and arranged to open into the patient's mouth for receiving said pin, characterized in that said pin is of one-piece construction comprising:

a flange (238) having first and second sides,

an externally threaded stem (236) projecting integrally from said first side of said flange and threadedly receivable in said threaded hole said anchor, said stem being of smaller width than said flange, and

a post (240) projecting integrally from said second side of said flange and including a portion (262, 264) of non-circular cross-section and longitudinally irregular profile, said post being of less width than said flange and adapted to have a dental prosthesis formed therearound.

23. A pin (234) adapted to carry a dental prosthesis and mounted in a preinstalled dental anchor in a patient's jaw, said pin being of one-piece construction and characterized by:

a flange (238) having first and second sides,

an externally threaded stem (236) projecting integrally from said first side and adapted to be threadedly received in the dental anchor, said stem being of smaller width than said flange, and

a post (240) projecting integrally from said second side and including a portion (262, 264) of non-circular cross-section and longitudinally irregular profile, said post being of less width than said flange and adapted to carry a dental prosthesis.

24. A method of drilling a bore of predetermined depth in a jaw bone of a patient so that a dental anchor can be installed therein, said method comprising the step of advancing a hollow head (320) of a trephine drill (322) longitudinally into the jaw bone to form a bore having a center core, characterized in that said advancing step is continued relative to a stop member (340) which is freely longitudinally slidably mounted on said drill until said stop member becomes sandwiched between the jaw bone (310) and a surface (344) movable with a motor housing carrying said drill, said stop member having a cross-section larger than a cross-section of said drill head and having a longitudinal length (H) of such dimension that when said length is subtracted from a longitudinal distance (X) between a front end of said drill head and said surface movable with said motor housing, the remainder (D‴) equals said predetermined depth.

25. A method of drilling a bore of predetermined depth (D‴) in a jaw bone of a patient so that a dental anchor can be installed therein, said method comprising the steps of drilling a longitudinally extending pilot hole (312) in the bone, installing in said pilot hole a bushing comprising first and second cylindrical portions (314, 316) such that said first cylindrical portion enters said pilot hole and said second cylindrical portion bears against the bone (310), inserting a hollow head (320) of a trephine drill in telescoping fashion over said second cylindrical portion such that said second cylindrical portion restrains said drill against sideways motion, and advancing said drill head longitudinally into the jaw bone to form a first core portion (332), removing said drill from said bore, breaking off and removing said first core position, and reinserting said drill head into said bore and advancing said drill head longitudinally to extend the depth of said bore while forming a second core portion (350) therein, characterized in that the advancement of said drill head along said second cylindrical portion occurs until an abutment shoulder (344) of said drill abuts an end of said second cylindrical portion to form a bore (323) of a desired first depth (D″), which is less than said predetermined depth, said bore containing a first core portion (332).

26. A dental trephine drill (410) of the type which cuts a bore having a central core (426A), said drill comprising a shank (412) having a fluid passage (416) extending longitudinally therethrough for conducting drilling fluid, a cutting head (414) disposed at a front end of said shank, said head being hollow and including a front end face (420) and a side face (422) extending longitudinally rearwardly from an outer periphery of said end face, said end face surrounding a central hole (424) adapted to receive a core (426, 426A) formed in bone tissue being cut, a plurality of end cutting edges (430) disposed on said end face and extending from said hole to said outer periphery of said end face, characterized in that said side face includes a plurality of longitudinally oriented grooves (438) extending rearwardly from said end face, a circumferentially trailing edge (440) of each groove defining a longitudinally extending side cutting edge at least a substantial portion of which being spaced at a constant distance from a rotary axis of said drill so as to cut a cylindrical bore, aperture means (452) in each groove communicating said grooves with the interior of said head for conducting drilling fluid into said grooves to cool said side cutting edges and bone.

27. A transparent sheet (510) carrying on a first side thereof a plurality of pictorial representations (512) of dental anchors of different sizes, there being a plurality of pictorial representations for each anchor size, characterized in that said pictorial representations are removable from said sheet such that a selected pictorial representation can be transferred to a receiver sheet by arranging said one side of said transfer sheet against the receiver sheet and rubbing on a second side of said transfer sheet opposite said selected pictorial representation.

_Fig. 1_

_Fig. 2_

Fig.3

Fig.4

Fig.5

80

62 · 36 A · 10 · 36 · 16 · 18 · 38 · 50 · 70 · 24 · 60 · 50

10 · 36 · 16 · 50 · 60 · 50 · 24

78A · 10 · 36 · 36 · 16 · 50 · 50 · 60

0 256 708

Fig.6

Fig.7

Fig.8

Fig.9

Fig.10

FIG.11
PRIOR ART

FIG.12
PRIOR ART

FIG.13
PRIOR ART

Fig.14

Fig.15

Fig.17

Fig.16

Fig.18

Fig.19

Fig.20

Fig.21

Fig. 24

Fig. 22

Fig. 23

FIG.25

218

210

222

FIG.26

232

218

220

210

228

226

224

FIG.27

220

233

210

230

FIG.28

246

240

234

250

250

242

248

238

236

244

220

FIG.28A

234

238

236

240

246

Fig.29

Fig.30

Fig.31

FIG. 32

FIG. 33

FIG. 34

FIG. 34A

FIG. 35

278

296

294

290

277'

258

260

*Fig.36*

276

278

*Fig.37*

292

274

290

238

236

299

292A

276

292

236

210

*Fig.38*

*Fig. 39*

*Fig. 40*

*Fig. 41*

*Fig. 43*

*Fig. 42*

*Fig. 44*

FIG.45

FIG.46

FIG.47

FIG.48

FIG.49

340
324
322
320
342
346
d

326
325
344
340
328
320
323 310
346
X

326
325
344
340
320
H
X
D''''

345 310
356 350
D'''

310 352

FIG. 50

FIG. 51

FIG. 52

FIG. 53

Fig.54

Fig.55

Fig.56

Fig.57

FIG. 58

FIG. 59

FIG. 60

3.5 mm DIAMETER

510

7 mm

3.5mm 7mm | 3.5mm 7mm | 3.5mm 7mm | 3.5mm 7mm | 3.5mm 7mm | 3.5mm 7mm

3.5mm 9.5mm | 3.5mm 9.5mm | 3.5mm 9.5mm | 3.5mm 9.5mm | 3.5mm 9.5mm | 3.5mm 9.5mm | 3.5m 9.5r

9.5mm

512

3.5mm 13mm | 3.5mm 13mm | 3.5mm 13mm | 3.5mm 13mm | 3.5mm 13mm | 3.5mm 13mm

13 mm

4.5 mm DIAMETER

512

7 mm

4.5mm 7mm | 4.5mm 7mm | 4.5mm 7mm | 4.5mm 7mm | 4.5mm 7mm

512

4.5mm 9.5mm | 4.5mm 9.5mm | 4.5mm 9.5mm | 4.5mm 9.5mm | 4.5mm 9.5mm | 4.5mm 9.5m

9.5 mm

512

4.5mm 13mm | 4.5mm 13mm | 4.5mm 13mm | 4.5mm 13mm | 4.5mm 13mm | 4.5mm 13mm

13 mm

5.5 mm DIAMETER

7 mm

5.5mm 7mm | 5.5mm 7mm | 5.5mm 7mm | 5.5mm 7mm | 5.5mm 7mm

520

5.5mm 9.5mm | 5.5mm 9.5mm | 5.5mm 9.5mm | 5.5mm 9.5mm | 5.5mm 9.5mm

9.5 mm

5.5mm 13mm | 5.5mm 13mm | 5.5mm 13mm | 5.5mm 13mm | 5.5mm 13mm

13 mm

514

516

7 mm

5.5 mm 9.5 mm | 5.5 mm 9.5 mm | 5.5 mm 9.5 mm

9.5 mm

512

5.5 mm 13 mm | 5.5 mm 13 mm | 5.5 13

510

514

512

5.5 mm 9.5 mm

518

514

5.5 mm
9.5 mm

518

512

FIG. 61

FIG. 62

510

7 mm

5.5 mm
9.5 mm

516

9.5 mm

514

5.5 mm
13 mm

5.5 mm
13 mm

5.5
13

13 mm

512A

514

5.5 mm
13 mm

512A

518

FIG. 63